(19) Europäisches Patentamt
European Patent Office
Office européen des brevets

(11) **EP 4 510 995 B1**

(12) **EUROPEAN PATENT SPECIFICATION**

(45) Date of publication and mention
of the grant of the patent:
**15.04.2026   Bulletin 2026/16**

(21) Application number: **23716630.1**

(22) Date of filing: **18.04.2023**

(51) International Patent Classification (IPC):
*A61K 8/14* (2006.01)          *A61K 8/55* (2006.01)
*A61K 8/63* (2006.01)          *A61K 8/68* (2006.01)
*A61Q 19/00* (2006.01)         *A61Q 19/08* (2006.01)

(52) Cooperative Patent Classification (CPC):
**A61K 8/14; A61K 8/553; A61K 8/63; A61K 8/68;**
**A61Q 19/00; A61Q 19/08;** A61K 2800/5922

(86) International application number:
**PCT/EP2023/060034**

(87) International publication number:
**WO 2023/203034 (26.10.2023 Gazette 2023/43)**

(54) **COSMETIC COMPOSITION CONTAINING A VESICULAR CONCENTRATE CONSISTING OF A WATER SOLUTION OF LIPID VESICLES FOR COSMETIC PRODUCTS, COSMETIC PRODUCTS CONTAINING SAID COSMETIC COMPOSITION AND METHOD FOR THEIR PRODUCTION**

KOSMETISCHE ZUSAMMENSETZUNG ENTHALTEND EIN VESIKELKONZENTRAT AUS EINER WÄSSRIGEN LIPIDVESIKELLÖSUNG FÜR KOSMETISCHE PRODUKTE

COMPOSITION COSMÉTIQUE CONTENANT UN CONCENTRÉ VÉSICULAIRE CONSTITUÉ D'UNE SOLUTION AQUEUSE DE VÉSICULES LIPIDIQUES POUR PRODUITS COSMÉTIQUES, PRODUITS COSMÉTIQUES CONTENANT LADITE COMPOSITION COSMÉTIQUE ET PROCÉDÉ POUR LEUR PRODUCTION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **20.04.2022   IT 202200007817**

(43) Date of publication of application:
**26.02.2025   Bulletin 2025/09**

(73) Proprietor: **Intercos S.p.A.**
**20123 Milano (IT)**

(72) Inventors:
• **RANDO, Pietro**
  **24060 PARZANIGA BG (IT)**

• **VALSESIA, Patrizia**
  **23885 CALCO LC (IT)**
• **DISTEFANO, Gaetano**
  **24127 BERGAMO (IT)**
• **GOLSTEIJN, Stefan**
  **6101 GW Echt (NL)**

(74) Representative: **Mittler, Enrico et al**
**Mittler & C. S.r.l.**
**Viale Lombardia, 20**
**20131 Milano (IT)**

(56) References cited:
**KR-A- 20110 073 495     KR-B1- 101 440 383**
**US-A1- 2003 118 616**

**Description**

The present invention relates to a cosmetic composition containing a concentrate of biomimetic lipid vesicles, used for preparing cosmetic products of different types.

Said cosmetic products are products for skin and hair care, and for decorating face, eyes, eyelashes, lips and body.

More particularly, the present invention relates to cosmetic products containing a concentrate of composite colloidal lipid vesicles (liposomes) containing active ingredients, wherein the structure of the wall of said vesicles comprises phospholipids, phytosphingosine, phytosterol, beta-phytosteryl sulfate and other derivates of phytosterol, ceramides and/or pseudoceramides (synthetic molecules mimicking the structure of natural ceramides), with the optional addition of fatty acids, humectants, emollients and other, and with the optional presence of active lipophilic molecules.

Moreover, the invention offers an original solution to the problem of preparing vesicles (liposomes) and to their introduction in a stable form inside cosmetic formulas.

As known, vesicles (or liposomes) are supramolecular structures made of phospholipids, which can be found everywhere in biologic systems, and that, by virtue of their amphiphilic structure, auto-assemble in aqueous environments according to a double layer wall structure, wherein their hydrophilic portion is in contact with the aqueous environment, while their hydrophobic portion is hidden inside said double layer.

In living organisms, said vesicles are made of a supramolecular shell or membrane made of a simple double layer of phospholipids and have the function of secretion (exocytosis), transport and internalization (endocytosis) of hydrophilic compounds, with an important role in intercellular communication.

Moreover, it also known that in living organisms vesicles can embed a variety of compounds inside the membrane, like membrane proteins and other lipophilic substances, which find a suitable environment inside said double layer.

Biomimetic lipid vesicles can be manufactured using synthetic phospholipids or phospholipids derived from the purification of phospholipids extracted from vegetal or animal sources.

It is known that the permeability of the vesicles and their mechanical stability depend on the composition of the lipid mix forming the membrane, and that said properties can be modulated through the use of lipids having different degrees of unsaturation or through the intercalation of sterols in the membrane.

Biomimetic lipid vesicles are microscopic structures comprising one or more lipid bilayers, arranged concentrically or tubularly. Different amphiphilic molecules were used in the literature in order to manufacture liposomes, while the method for their preparation can be adapted in order to control their dimensions and morphology. Active molecules can be encapsulated in the aqueous space or intercalated in the double lipid layer; the exact position of the active molecules in the vesicles depends on the physical-chemical structure and on the lipid composition. In literature, biomimetic lipid vesicles have shown a great potential as systems for releasing active ingredients for personal care. A wide array of molecules, including peptides and proteins, has been incorporated in biomimetic colloidal lipid vesicles through different preparation systems. Thanks to their high degree of biocompatibility, they are one of the most efficient systems for favouring the cutaneous delivery of actives and skin treatment.

In synthetic vesicles, the organization of the double layer can be single (so-called SUV/LUV, Small/Large Unilamellar Vesicles) or multiple (SMV/LMV Small/Large Multilamellar Vesicles), forming unilamellar or **multilamellar** vesicles, respectively, small or big, provided with a more or less thick shell, or of smaller or bigger dimensions.

The dimensions of synthetic lipid vesicles depend on their manufacturing methods. At the state of the art, there are provided different methods for manufacturing synthetic lipid vesicles, mainly through the use of ultrasounds, extrusion through porous membranes, hydration of thin phospholipidic films, or through micro-fluidic techniques. Such methods do not allow to obtain a high density of vesicles, but allow their precise dimensioning, and therefore are the methods of choice in the academic and pharmaceutical field.

Especially in the pharmaceutical and biologic context, the ability of liposomes to penetrate through tissues in order to release their content inside cells is of paramount importance. Inside cells, the active ingredient contained in the vesicles can perform its pharmacologic action. The penetration through tissues is promoted through the manufacturing of liposomes provided with nanometric dimensions (diameter < 100 nm) and therefore such formulations are extremely precise and calibrated for such aim. They are administered in controlled scenarios, and undergo severe tests. In fact, they are injectable formulations (like in the case of so-called mRNA vaccines) or are used, through their interaction with cell membranes, for the in-vitro internalization of genetic materials.

Nowadays, consumers are more and more fond of products for personal care, which make daily life healthy and pleasant, improve social relationship and lead to personal well-being. For these reasons, today the quality of the new products for personal care is higher and higher, thanks to the introduction of new sophisticated and performing ingredients. The trend toward the preparation of products with attention to the environmental sustainability and ingredients renewability is especially strong. In this regard, phyto-derived ingredients and compounds, taken from plant world, are especially preferred.

This consumer need and new scientific knowledge lead the Applicant to study and develop an innovative project for preparing cosmetic products containing biomimetic vesicles.

In the cosmetic world, the interest in said vesicular systems derives from the fact that, similarly to what occurs inside living organisms, said vesicles can act as efficient carriers of hydrophilic and lipophilic active molecules. From literature, their capacity to encapsulate hydrophilic compounds inside their aqueous core is known, as well as their capacity to intercalate lipophilic compounds in their lipid bilayer shells. Moreover, it is known that the compounds forming the vesicles/liposomes can be chosen among the substances of biologic derivation, mimicking the lipid components present on the epidermis. They can integrate and improve epidermis barrier properties to external agents and against dehydration.

The feature of encapsulation of active components can be exploited in the creation of personal care products, especially skin care products. In fact, on the market there are provided, and in literature there are described, products featuring the presence of liposomal vesicles in face creams and other topical products. Such products contain, among other, differently functionalized phospholipids, ceramides, fatty acids and sterols, which are essential components for preparing liposomes; nonetheless, the existence and the integrity of the vesicular structure in the cosmetic product is not demonstrated. As the preparation of vesicles is a process requiring the total control of the composition, temperature and stirring strength to which the phospholipidic layers are exposed, their in-situ formation in the final composition is far from being ensured. In a market context wherein the claimed properties of the cosmetic and personal care products, especially high-end products, must be demonstrated and supported by scientific evidence, companies need to be provided with methods and techniques capable of enforcing such claims.

In fact, vesicular structures are structures maintained by supramolecular weak interactions, and can easily be destructured by processing energy, temperature, use of emulsifiers and solvents, leading to different multiphasic systems, like e.g. oil-in-water emulsions.

The scenarios in the cosmetic field are distinct from the pharmaceutical ones for the need to create pleasant and at the same time functional textures, by modulating many ingredients that are combined through the total mastery of often heterogeneous productive processes. Moreover, the methods described in academic and patent literature are suitable for the manufacture of liposomes and vesicles provided with nanometric dimensions (<100 nm), featuring an elevated in-vivo tissue penetration. Such feature is counterproductive in the dermo-cosmetic field, where structures intentionally manufactured with dimensions smaller than 100 nm are considered nanomaterials, that is often a non-desired feature that can worry the user.

The cosmetic formulator must face a wide formula variability, while the manufacturing of formulas containing intact liposomes/vesicles is a challenge.

E.g., the use of traditional secondary emulsifiers mixed with the vesicles is not compatible with the phospholipid system, in that it destroys its order and compromise its efficacy. One of the possible formula strategies is inserting vesicles into polymeric gels, in order to provide the final product with optimal features, like ease of use and sensory agreeableness. These composite structures are provided with peculiar features, among which an increased stability of vesicles in macromolecular systems, giving a higher resistance than free vesicles in a solution. Moreover, when an active molecule is placed in the core of the vesicles and the vesicles are included in a gel web, both the web and the double layer preserve the different active ingredients at their best during transport. Finally, the controlled release of the active ingredients over time reduces to a minimum the problem of the "burst release", i.e. the overdosing of the active ingredients due a too rapid release.

KR 101440383 B1 describes a cosmetic composition containing osmocells in form of vesicles, comprising a lipid complex of squalene and a human adipocyte stem cell media extract. The osmocell are obtained by emulsifying squalene, phospholipids, phytospingosine, ceramide, cholesterol, phytosterol, beta-sitosterol at high pressure to form a gel-type lipid complex. At room temperature, this complex is added to an aqueous solution comprising said extract in order to obtain vesicles comprising the extract complexed with squalene inside a closed double layer structure. Said osmocells substantially comprise a lipid composition forming a cellular membrane prepared in order to stabilize a human body fat stem cell culture extract which is unstable to heat, and to facilitate transdermal absorption (paragraph 0026). The osmocells according to this document are characterized by dimensions ranging 20 to 500 nm, preferably 50 to 250 nm, and most preferably 100 to 200 nm, 120 nm on average. According to the document, when the average droplet size of osmosis is more than 500 nm, skin permeation and formulation stability are very weak (paragraph 0029).

Object of the present invention is to manufacture cosmetic products containing biomimetic vesicles functional for skin care, wherein the integrity of vesicles is preserved and their beneficial activity on epidermis health is shown.

Said object is obtained by creating a cosmetic composition containing a vesicular concentrate and using said cosmetic concentrate vesicular composition for the preparation of cosmetic products according to claim 1.

Said vesicles are formulated specifically in a processing step wherein only the ingredients needed for preparing them are combined and processed according to modes ensuring the formation of vesicles with high yield and high concentration. Successively the vesicles are combined with other cosmetically acceptable and compatible ingredients, through a process suitable for preserving their identity.

The wall of vesicles is preferably made of phospholipids (including, but not limited to: phosphatidylcholine, phosphatidylethanolamine, phosphatidylserine, phosphatidylinositol, phosphatidylinositol phosphate, phosphatidylinositol bisphosphate, phosphatidylinositol trisphosphate etc., each with different degrees of saturation/unsaturation) monoacyl derivatives of phospholipids (including, but not limited to: monoacylphosphatidilcholine), steroid derivatives (including, but not limited to: phytosterol, beta-sitosterol, phytosteryl isostearate, beta-phytosteryl sulfate etc.), ceramides and similar (sphingosine acylate and/or "pseudoceramides"), sphingosine and similar, aliphatic oils (including, but not limited to: squalane), fatty acids (including, but not limited to: oleic acid), fatty alcohols (including, but not limited to: octyldodecanol), antioxidants, preservatives, and optionally active lipophilic molecules. Fatty alcohols and steroid components can be optionally modified with silane functional groups (including, but not limited to: triethoxysilane functional groups).

A method used for obtaining a cosmetic product according to the present invention is defined in claim 15.

In particular, the vesicular concentrate of the cosmetic composition according to the present invention, can be obtained by bringing to melted state (liquid crystalline state) the components of the vesicle (PHASE A) in a humectant of vegetal origin (including, but not limited to: glycerol, diglycerol, isosorbide, butylene glycol) up to obtaining a homogeneous monophasic system, and introducing, at a temperature higher than the solidification temperature of the lipid phase, the aqueous phase into the system in order to organize the liquid-crystalline lipid phase into multilamellar vesicular structures.

The active ingredients (PHASE B) that can be inserted in the vesicular concentrate are preferably of vegetal origin, including, but not limited to: stearyl glycyrrhetinate, magnolia acuminata bark extract, betula alba bark extract, centella asiatica leaf extract, jania rubens extract, tocopheryl acetate, hydroxypinacolone retinoate, linolenic and linoleic acids, ascorbyl tetraisopalmitate, 3-o-ethyl ascorbic acid, niacinamide, fructooligosaccharides, beta vulgaris (beet) root extract, tranexamic acid, sophora japonica extract, hexylresorcinol, undecylenoyl phenylalanine, salicylic acid, salix alba bark extract, zinc pyrithione, brassica campestris (rapeseed) flower extract, candida bombicola/canola oil ferment extract, undecylenic acid and derivatives, climbazole, quillaja saponaria bark extract, cupressus sempervirens fruit extract, arctium majus root extract, 10-hydroxydecanoic acid, sebacic acid, zinc pca, caprylic acid, ficus carica (fig) fruit/leaf extract, urtica dioica extract, hydrolized yeast protein, kigelia africana fruit extract, ginkgo biloba leaf extract, basilicum hairy root culture extract, caffeine, neoruscogenin, ruscogenin, betaine, avena sativa, zanthoxylum alatum fruit extract, brassica campestris (rapeseed) sterols, epilobium angustifolium flower/leaf/stem extract, hydrogenated starch hydrolysate, ascophyllum nodosum extract, asparagopsis armata extract.

The concentration of the lipid ingredients of the vesicles can range 15 to 40% by weight of the vesicular concentrate.

The aqueous phase contains water and comprises one of the following: humectants (including, but not limited to: glycerol, diglycerol, isosorbide, butylene glycol), salts, preservatives, acidity regulators (including, but not limited to: lactic acid, citric acid, sodium hydroxide, arginine), rheology modifiers, hydrophilic active substances, and any other hydrophilic cosmetically acceptable compound.

The system is then brought under the solidification temperature of the lipid phase and it looks like a material with pasty texture having the rheologic features of a pseudoplastic fluid (shear thinning, or that becomes more fluid under the action of increasing shear rates), or of a viscoelastic solid (elastic modulus higher than the viscous modulus at low strains with inversion between the moduli at high strains). The dimensional distribution of the vesicles so prepared ranges 0.6-1.0 microns, with average dimensions of 0.8-1 $\mu$m (determined through laser diffraction) and is depending on the intensity of the shear forces applied during the manufacturing step of the concentrate.

In particular, with reference to Example 1 and following examples, the vesicular concentrate can be obtained by inserting the ingredients of PHASE A one at a time into a jacketed mixer provided with turbine (rotor-stator), blade mechanical stirring at room temperature and wiper. The mix is then mechanically stirred at room temperature up to obtaining a homogeneous smooth dispersion of the components. PHASE A is gradually heated with increments of 10°C in a period of 1h up to the temperature of 80-100°C in order to allow the fusion of solid components, obtaining a translucid dispersion provided with a pasty texture and slightly yellowish. The PHASE A is then brought to 60°C continuing the mechanical stirring, while the PHASE B is gradually added to the mixer keeping the system at constant temperature. Once the PHASE B addition is completed, the composition is kept under stirring for 30 minutes. Finally, a turbulent stirring is applied through the use of the turbine and the temperature gradually reduced up to room temperature, obtaining a concentrate having the appearance of a white/yellowish paste. The vesicular concentrate so obtained can be diluted in water and other ingredients in order to obtain the formulas of Example 4 and following examples.

Cosmetic products are obtained by combining the vesicular concentrate with water and hydrophilic ingredients like humectants (including, but not limited to: glycerol, diglycerol, isosorbide, butylene glycol), salts, preservatives, acidity regulators (including, but not limited to: lactic acid, citric acid, sodium hydroxide, arginine), rheology modifiers (including, but not limited to: polyquaternium-37, polyacrylates, polysaccharides, xanthan gum, bentonite), hydrophilic active substances, and any other hydrophilic cosmetically acceptable compound. Moreover, cosmetically acceptable lipophilic ingredients can be optionally integrated in the formula, like oils, waxes, silicones and derivatives. The concentrate is contained in the cosmetic product for a 0.1-99.99% by weight of the final product, and is combined with the other cosmetic ingredients in conditions wherein the integrity of the vesicles is not compromised.

Summarizing, the method for obtaining a cosmetic product according to claim 1 comprises the following steps:

- preparing vegetal or biomimetic vesicles of vegetal origin having dimensions ranging 0.6 - 1.0 micron with walls including a lipid phase with the above-mentioned ingredients by performing the following sub-steps:

  o preparing a composition containing a vesicular concentrate through the fusion of the walls of said vesicles up to the liquid-crystalline state, so as to obtain a homogeneous monophasic system;
  o introducing an aqueous phase into said vesicular concentrate composition at a temperature higher than the solidification temperature of the lipid phase of the vesicles;
  o cooling the vesicular concentrate composition under the solidification temperature of the lipid phase of the vesicles up to the obtainment of a pasty material having the rheologic features of a pseudoplastic fluid or a viscoelastic solid with a dimensional distribution of the vesicles ranging 0.6-1.0 microns;

- combining the vesicular concentrate composition having pasty texture with water and other cosmetic ingredients up to obtaining a final cosmetic product containing a desired percentage by weight of said vesicular concentrate.

It is worth to point out that the compositions prepared according to the present invention preferably contain only raw ingredients of vegetal origin.

Among cholesterol-like molecules, phytosterols are a generic term indicating molecules of vegetal origin; to this category belongs beta-sitosterol sulphate, that is chemically identical to cholesterol sulphate of animal origin, and also phytosterol isostearate, that it is an ester.

The use of the sulphate form of phytosterols gives to the present composition features different from those of the plain cholesterol, both of animal or vegetal origin. For further information, reference is made to the review of Strott and Higashi Cholesterol sulfate in human physiology: what's it all about? Journal of Lipid Research, 2003(44):1268-1278. The review explains that cholesterol sulphate is a component of cell membranes and can be found in human plasma. Surprisingly, it shows regulatory functions, suggesting a similarity to compounds categorized as hormones. In particular, it plays a role in the differentiation of keratinocytes and in the development of epidermal barrier. It is believed that cholesterol sulphate plays a significant role in the proper functioning of the stratum corneum and in the normal sloughing of dead cells. A reduction in cholesterol sulphate content in the stratum corneum decreases the stability of bilayers and reduces the fraction of lipids arranged in liquid lateral packing, which results in a reduction in the elasticity of the lipid phases, preventing proper corneocyte lipid lamellae orientation. Cholesterol sulphate has demonstrated a regulatory influence on the transcriptional activity of specific keratinocyte genes.

As explained in the introduction, in the art using liposomes for transporting active molecules and helping their penetration into cells is known; KR 101440383 B1, too, discloses osmocells containing a human adipocyte stem cell media extract.

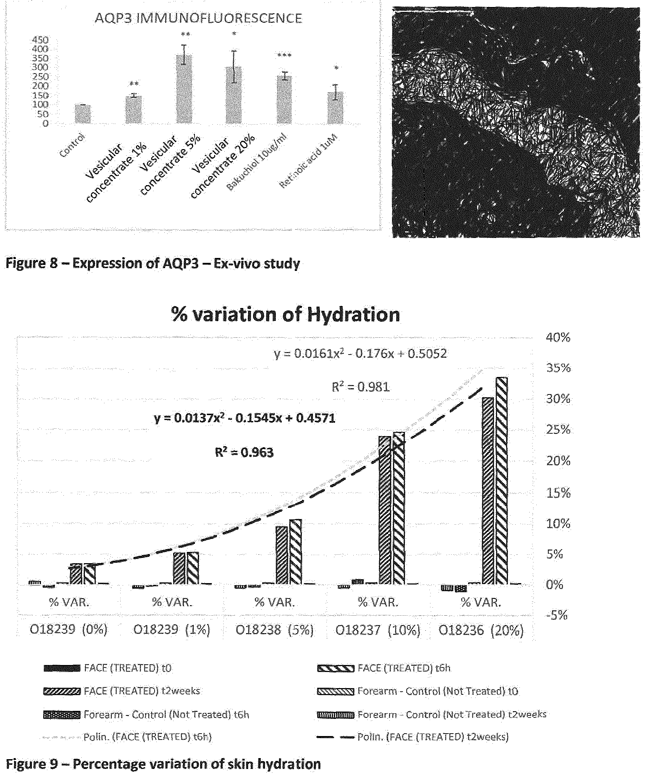

Surprisingly, in the skin application of the present invention, it was noted that the improvement of skin conditions can be observed already after 6 hours (see comment to Figure 9), which is inconsistent with an action due to an active ingredient contained inside the vesicles. Moreover, it is also interesting that a week after stopping the application of the cream according to the present invention, hydration goes back to the initial values (see points 5.1.2 and 5.1.3).

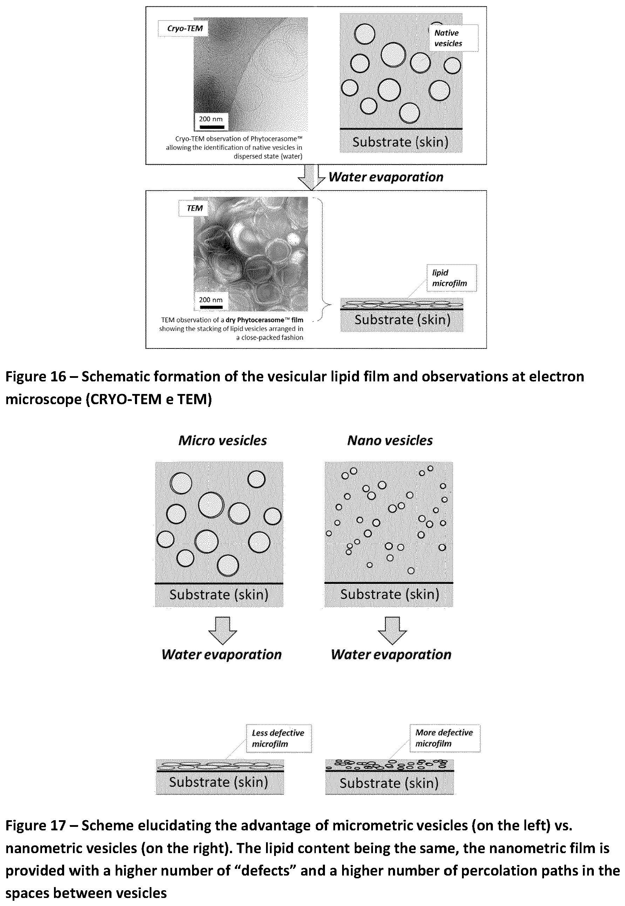

Such observations can be explained only with the consideration that the vesicular concentrate (and the formulas containing it in different percentages) can deposit a continuous film made of the non-volatile components (the lipid shell of vesicles), having a lateral dimension in the order of a micron. Such evidence is substantiated by organoleptic observations on the texture of the product (the feeling of an emollient and nourishing film) and by instrumental observations (TEM electronic microscope images of the vesicles after deposition and evaporation of the aqueous components, see Figures 16 and 17). The vesicular structure is maintained in the presence of the active ingredients; the filming and nourishing capacity remains also when active ingredients are present, and allows an immediate effect that can be observed within a few hours. To the short-term filming effect, the long-term effect is added, deriving from the repeated exposition to the specific active ingredients improving the functions of the concentrate on a time scale compatible with the modification of cellular metabolism (the two-four weeks of the clinical test).

Moreover, the present invention relates to the preparation of cosmetic water-based products for the decoration of face, eyes, eyelashes, lips and body, containing said vesicular concentrates, and cosmetically acceptable pigments.

Finally, the present invention relates to the manufacturing of cosmetic powders obtained by combining the vesicular concentrate with powders and removing the aqueous phase with an additional evaporation process.

One of the main functions of the skin is protecting the organism from environmental factors (e.g. ultraviolet radiations, microorganisms, physical and chemical agents). The whole surface of our skin is covered with a basal lipid layer forming a protective layer. This lipid layer, called skin hydrolipidic film, is made mainly of lipids (95%) and has an acid pH (4.9).

The integrity of the hydrolipidic film is paramount for maintaining a soft and hydrated skin, protecting the body from external microorganisms, reducing the risk of infections, regulating the absorption and the excretion of substances through the skin.

In fact, said lipids ensure a reduced water dispersion, a reduced dispersion of electrolytes, a natural defence against microorganisms, and a cementing action for keratinocytes.

The efficacy of formulas containing the vesicular concentrate was shown in the following fields: skin barrier enhancement, skin rejuvenation, anti-age (or pro-age) activity, increase in skin hydration, immediate effect, improvement of skin elasticity and skin firmness, reduction of wrinkle visibility, stimulation and increase of the production of collagen I, stimulation and increase of the activity of aquaporin-3 channels, retinol-like activity.

The efficacy is shown in the examples of formulation of vesicular concentrates and derived cosmetic products, which are disclosed at the end of the present description.

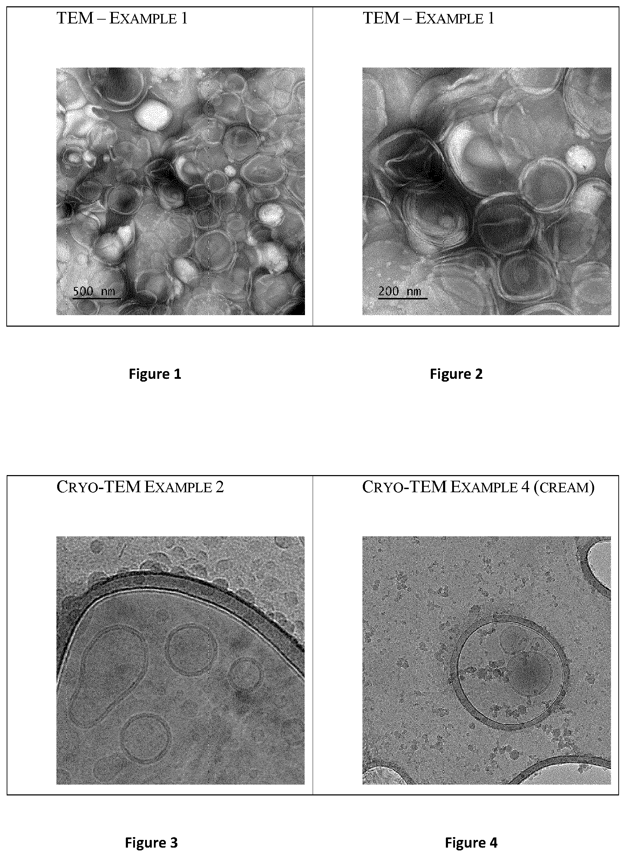

Figures 1 and 2 show the images, obtained through Transmission Electron Microscopy (TEM), of the vesicular concentrate of Example 1. The images show the multilamellar morphology and the characteristic dimensions of the vesicular structures generated through the present invention (on the left: scale bar 500 nm, on the right: scale bar 200 nm).

Figure 3 shows the image, obtained through transmission electron microscope on frozen samples (cryo-TEM), relating to the vesicular concentrate of Example 2, showing intact vesicles.

Figure 4 shows the image, obtained through transmission electron microscope on frozen samples (cryo-TEM), relating to the cream formula of Example 4 with the vesicular concentrate of Example 2 10% by weight. Vesicular structures are preserved.

In particular, experimental testing of Example 1 was performed, which is summarized hereunder.

## 1) Evaluation of the efficacy of the vesicular concentrate according to Example 1

The efficacy of the vesicular concentrate according to Example 1 was shown by a strategy of wide-ranging tests, using a fairly complete set of *in-vitro, ex-vivo* and *in-vivo* (clinical test) tests.

## Activity of the vesicular concentrate

According to the observations, the vesicular concentrate according to Example 1, when used in cosmetic formulations, can be considered effective in:

- skin barrier enhancement
- protection and restoration of human skin barrier
- skin rejuvenation
- anti-age / pro-age activity
- immediate increase of skin hydration
- improvement of skin elasticity and skin firmness in a short time
- reduction in wrinkles visibility
- improvement of overall skin conditions
- general immediate and perceivable effects
- highly effective retinol-like activity.

## Efficacy of the vesicular concentrate

In order to show the efficacy of the vesicular concentrate according to Example 1 specific endpoints where used, linked to skin barrier and to skin rejuvenating activity.

## Efficacy linked to the activity of skin barrier / renewal /rejuvenation

Different endpoints were selected for *in-vitro* studies on cell cultures and *ex-vivo* studies on human skin explants: production of Aquaporin-3 (AQP3) and synthesis of Pro-Collagen I.

## Evaluation of non-cytotoxic concentrations to be used on cell cultures: MTT on keratinocytes (HaCaT)

A preliminary test of cytotoxicity (MTT assay on HaCaT cells 48h treated with increasing concentrations of vesicular concentrate) was performed for selecting the highest non-cytotoxic dose of vesicular concentrate (0.011% and 0.017%) to be used in the following *in-vitro* studies.

**Type I Collagen**

Collagen is the main structural protein of the extracellular matrix; it can be found in all the different connective tissues of animals. Type I collagen is one of the most represented collagens (the most abundant) in derma and is the "scaffolding" protein essential in imparting strength and structure to skin.

The production of Collagen I decreases with age, so reducing the smoothness and elasticity of skin, and increasing wrinkle formation.

Pro-collagen is a precursor of collagen. Type I Pro-Collagen is the new collagen synthetized in derma. Increasing the synthesis of collagen fights skin aging and improves skin health.

**Production of Type I Collagen - In-vitro evaluation on cell culture using two different concentrations of vesicular concentrate according to Example 1**

This test monitored the synthesis of new Collagen I through the quantification of type I Pro-Collagen using specific antibodies through an ELISA test on a cell culture made of a monolayer of normal human dermal fibroblasts (NHDF).

Two water dispersions at 0.01% and 0.017% of vesicular concentrate according to Example 1 were applied to a culture medium for 24 hours. The peptide TGFβ (Transforming Growth Factor Beta 1) - the natural growth factor involved in tissue repair - was used as positive control. Three independent tests were performed, each of which was performed in triplicate.

**RESULTS**

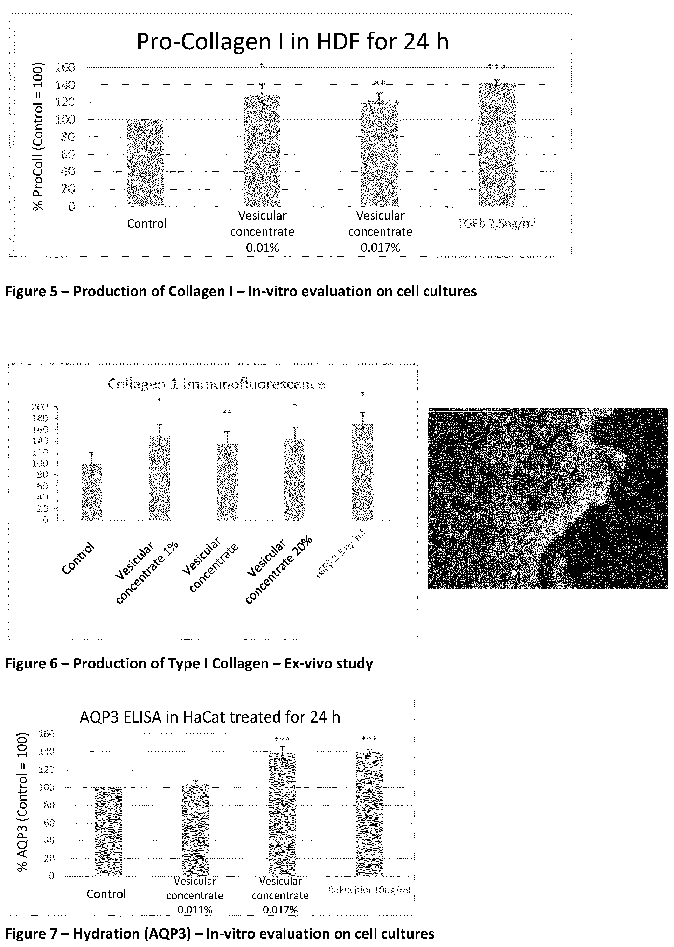

The vesicular concentrate significantly increased the synthesis of Pro-Collagen I of 20% at both tested dilutions, as shown in Figure 5 (*=p value 0.05; **= p value 0.01; ***= p value 0.001), showing the production of type I Collagen and in particular its in-vitro evaluation on cellular cultures.

**Production of Type I Collagen - Ex-vivo evaluation on cell culture using three different concentrations of vesicular concentrate according to Example 1**

In order to confirm the effects of the vesicular concentrate on the stimulation of the synthesis of Type I Collagen, biopsies derived from human skin explants were treated with three different concentrations (1%, 5%, 20%) of vesicular concentrate in glycerol. The control sample contained glycerol only, while as positive control TGFβ (Transforming Growth Factor Beta 1) was used, the natural factor involved in tissue regeneration.

The abundance of Type I Collagen was detected through ImmunoHystoFluorescence and the intensity of fluorescence calculated through ImageJ® software. Three independent experiments were performed, each performed in triplicate.

**RESULTS**

As shown in Figure 6 (*=p value 0.05; **= p value 0.01), the vesicular concentrate increased the production of Collagen I of 40%, even at the lowest tested concentration (1%). This effect was not dose-dependent, and is comparable to the result generated by the positive control.

In Figure 6:

**On the left:** the chart shows the percentage increment in the production of Type I Collagen with respect to the control blank sample (set as 100%) and to the positive control TGFβ.
**On the right:** image in immunofluorescence of a section of skin explant, localizing the most of Type I Collagen of new formation in the higher layer of derma, near to epidermis.

Therefore, it can be considered that - both in vitro and ex-vivo - the vesicular concentrate, even in low percentages, is shown to significantly stimulate the synthesis of new collagen, by monitoring the production of Type I Pro-Collagen.

**Aquaporin 3 (AQP3)**

Aquaporin 3 (AQP3) is the protein product of human gene AQP3. It is expressed in many tissues, such as skin, wherein it acts as membrane transporter of water and glycerol. It is one of the most important channels for water and glycerol in epidermis, regulates skin hydration and therefore maintains its good healthy balance.

Aquaporins (AQPs) are vital for maintaining water homeostasis; Aquaporin 3 channels (AQP3) - highly expressed in the basal layer of keratinocytes in normal skin - are the most important passive transporters of water and glycerol in epidermis; they regulate skin hydration by balancing it, and therefore, they maintain skin good health and functionality (e.g. elasticity). A marked decrease in the dermal water content and skin elasticity was observed in mice wherein the expression of AQP3 had been suppressed.

Therefore, AQP3 plays an important role in maintaining skin hydration and correct functioning.

**Production of AQP3 - IN-VITRO evaluation on cell culture using two different concentrations of vesicular concentrate according to Example 1.**

In this test, the efficacy of the vesicular concentrate in increasing the expression of Aquaporin-3 against the known active principle Bakuchiol used a positive control was evaluated.

Bakuchiol is a known natural active principle, provided with many cosmetic properties (anti-wrinkle, efficacy on acne-prone skin, stimulating the production of elastin, mitigating dark spots), making it a natural alternative safer than retinol, with similar anti-age benefits. In this test Bakuchiol was chosen as positive control for its capacity of stimulating the synthesis of AQPS as shown in literature (DOI: 10.1111/ICS.12117).

Two water dispersions (0.011% and 0.017%) of vesicular concentrate were applied for 24 hours on cell cultures made of a monolayer of human keratinocytes (Human Keratinocytes - HaCaT).

The quantity of expressed AQPS was evaluated through specific antibodies, using ELISA plates. Three independent experiments were run, each of which was performed in triplicate. The results were calculated as average.

**RESULTS**

The vesicular concentrate (Figure 7) showed a very good activity, increasing AQP3 in both the tested dilutions (0.010% and 0.017%) - with a dose-response model - obtaining an effect very similar to Bakuchiol in its usual concentration of use.

The histogram shows the percentage increase of AQP3 in comparison with the blank control (100%) and Bakuchiol as positive control (***= p value $\leq$ 0.001%).

**Production of AQP3 - EX-VIVO evaluation on cell culture using three different concentrations of vesicular concentrate according to Example 1.**

In order to confirm the stimulation of AQP3 by the vesicular concentrate - resulted from the above-described in vitro test - biopsies deriving from human explants were treated with topical application of three different concentrations (1%, 5%, 20%) of vesicular concentrate (Example 1) in glycerol. The expression of AQP3 was detected through ImmunoHistoFluorescence. Three independent experiments were run, each in triplicate, and the result was calculated as average. Retinol and Bakuchiol - known as activators of AQP3 - were selected as positive controls.

**RESULTS**

The chart of Figure 8 (*= p value p 0.05; **= p value 0.01; ***= p value 0.001) shows the intensity of the fluorescence detected by ImageJ® software: the vesicular concentrate at all the tested concentrations significantly increased the production of AQP3 channels. Even more interesting, the 5% vesicular concentrate increased AQP3 more than three times than retinol and nearly twice more than Bakuchiol.

In Figure 8:

**On the left:** the graph shows the percentage increase of AQP3 vs. control blank (set as 100%) and the two positive controls, Bakuchiol and Retinoic Acid.

**On the right:** images of ImmunoHistoFluorescence of sections of skin explants localizing AQP3 water channels.

From the in-vitro and ex-vivo studies on Aquaporin 3 channels the following can be deduced:

- The vesicular concentrate significantly increased the Aquaporin 3 channels in in-vitro and also in ex-vivo studies;
- On this parameter, the vesicular concentrate was much more effective than the well-known AQP3 activators Retinol and Bakuchiol.

**Clinical studies on the vesicular concentrate system**

Based on the excellent results of the in-vitro and ex-vivo experiments, a test strategy was established for the clinical evaluation of the vesicular concentrate.

The specific study endpoints were chosen in order to evaluate the anti-age activity of the vesicular concentrate, in particular focusing on its efficacy as skin barrier and skin rejuvenation.

The hereunder summarized results were confirmed by two different complete studies:

- a first study investigated the functionality of increasing percentages of vesicular concentrate (Example 1) in a basal formula of face cream;
- a second study explored vesicular concentrate systems provided with different quantities of their constituent components, so as to investigate the flexibility of the vesicular concentrate, to be considered more as a platform than as a cosmetic complex active ingredient made of a specific formula.

**4.1 Clinical study of the vesicular concentrate according to Example 1 in increasing percentage concentrations**

In this first study, five formulas of a basal face cream were prepared, containing increasing percentages of vesicular concentrate: 0% (placebo), 1%, 5%, 10%, 20%. The clinical evaluation test was conducted on 50 volunteers (age 40-65, females) subdivided into 5 groups of 10 volunteers; one of the five formulas was assigned to each group.

The first time, the product was applied on their face in the clinical centre, under controlled conditions; successively, the volunteers continued the use of the product applying it twice a day (morning and evening) for a period of 4 consecutive weeks (28 days).

In order to evaluate the anti-age efficacy of the vesicular concentrate, in particular its effects on the skin barrier and its skin renewing/rejuvenating activity, three different evaluation were chosen:

**4.1.1 Hydration (through corneometry evaluations),**
**4.1.2 Skin elasticity (through cutometry),**
**4.1.3 Wrinkle visibility (through digital images analysis).**

The instrumental measures were performed on the face of volunteers treated with the product (test area) and on a not treated area of the forearm (control area). The measurements were performed at different times (short term: 6 hours after the first application; medium term: 14 days; long term: 28 days) and compared to the basal values acquired at time 0, according to the scheme in the following table:

|  | HYDRATION test | ELASTICITY test | WRINKLE VISIBILITY test |
|---|---|---|---|
| tB - Basal | X | X | X |
| t6h - 6 hours from application | X |  |  |
| t2w - 2 weeks of application | X | X | X |
| t4w - 4 weeks of application |  | X | X |

The measures at 2 and 4 weeks were performed after about 4 hours since the last application of the product.

Given the influence that temperatures and relative humidity have on the skin, the volunteers were kept for at least 20 minutes in controlled conditions (range: $22 \pm 2°C$ and $50 \pm 5\%$ respectively) before the measures.

**4.1.1 Skin hydration** (evaluated through corneometry)

Skin hydration is a direct general parameter allowing to overall control skin good condition and functionality. Any alteration of skin barrier - due to physical, chemical or aging factors - leads to a decrease in the skin hydration level.

This study evaluated the hydrating efficacy of the vesicular concentrate according to Example 1 through instrumental measures of the skin hydration condition (corneometer CM 825 - Courage + Khazaka) after a single application and after repeated daily applications for 14 days. The collected data were statistically evaluated through average comparisons with two-tailed t-test.

**RESULTS**

Figure 9 shows a graphical representation of the results, showing the percentage variations of skin hydration values measured for the treated and not treated areas at different times, compared with their respective basal values at time 0.

The graph shows the percentage variations of skin hydration values after a single application (t6h) and after two consecutive weeks of double daily application of the test products (t2weeks), compared with the respective basal values (tb) in the treated area (face) and in the control not treated area (forearm) at the same measurement times.

The quadratic correlation function calculated for the values of the treated area after 6 hours and after 14 days is indicated too, with its respective equations.

The vesicular concentrate was shown to be effective in significantly hydrating the external layers of the skin: the average hydration levels of the face areas treated with the vesicular concentrate at all concentrations were significantly increased ($p < 0.05$) in comparison to basal values, 6 hours after a single application and 2 weeks after the daily application of the test products; no significant variation was detected in the control not treated area.

The good increase of the skin hydration value reached after just one application remained constant after two weeks of product application: this shows the immediate benefit of the vesicular concentrate, with its capacity of "blending" with skin and of creating a sort of unique "shield" that physically protects skin: in fact at the two different reading times (6 hours and 14 days) nearly identical hydration value were detected in all the groups - with no cumulative effect - and this indicates that the vesicular concentrate physically interacts with skin, in lieu of interacting with it metabolic system.

This effect is also confirmed by the practically linear correlation of the dose-effect response detected for the different vesicular concentrate tested percentages (1%, 5%, 10%, 20%): the reached hydration levels are directly proportional to the quantity of vesicular concentrate for both series (measurements after 6 hours and measurements after 14 days). [The data correlate best with a quadratic distribution ($R^2 = 0.9813$ and $R^2 = 0.9632$), but are very near to a linear distribution as the quadratic term coefficient is negligible in both correlations (ref: Figure 9)].

No minimal dose was detected for triggering the effect, nor a plateau was reached in the tested interval of concentrations.

These results on hydration were all confirmed by the second study, described below, that had such evaluation among the considered endpoints.

Therefore, the vesicular concentrate was shown effective in significantly hydrating the more external skin layers at all the tested percentages.

- Its efficacy is immediate: the good increase of the hydration level is reached after just one face application of the cream containing said vesicular concentrate (measurements after 2 hours and 6 hours) and remains constant for the whole period of application of the product on the skin. This result accounts for the immediate benefit given by the vesicular concentrate, with its capacity of creating a sort of unique "microfilm on skin" that physically protects it - because it interacts and blends with it, instead of interacting with its metabolism. In fact, skin hydration goes back to its initial basal value 7 days after the suspension of the use of all the different cosmetic products containing the vesicular concentrate under investigation.
- This effect is confirmed also by the excellent linear dose-response correlation: the increased hydration values are directly proportional to the tested quantities of vesicular concentrate (1%, 5%, 10%, 20%).
- It was detected that no minimal quantity of vesicular concentrate was needed to trigger its hydrating efficacy, nor a plateau was reached beyond a given dose.

**4.1.2 Skin elasticity: compactness and elastic properties** (with cutometry measurements)

In the same study conditions, the efficacy of the vesicular concentrate according to Example 1 in improving skin compactness and elasticity was tested.

Aim of the study was evaluating the efficacy of face creams containing increasing quantities (0% -placebo; 1%, 5%, 10%, 20%) of vesicular concentrate in improving the elastic properties of skin after repeated daily applications, for a total period of 4 weeks, in comparison with a not treated area (forearm).

The method was based on the instrumental evaluation of the capacity of the skin to go back to its initial position after suction performed in standard conditions. The principle of the measurement consists in suctioning the skin in the test area, obtained through a defined depression applied via a circular aperture (2mm diameter) of the instrument probe (Cutometer MPA 580 - Courage+Khazaka). An operative mode with a low and constant pressure level was applied: the skin is suctioned into the probe by the uniform vacuum applied for a specific time, then goes back to its original position when pressure is released. The experimental conditions were:

- Suction time = 1 sec;

- Release time = 1 sec;
- Pressure = 450 mbar

The measurement was repeated for 3 consecutive cycles.

The creams were applied on their face by the volunteers: the volar area of the forearm was used as not treated control. In order to ensure that measurements were taken always in the same place in the different times, a reference mask was used both for the treated and the not treated areas. The measurements on face were performed in the malar area. In order to randomize use conditions, measurements were performed in the malar and volar right areas on 25 volunteers, and in the left areas for the remaining 25 volunteers.

Measurements were performed before applying the product (basal reading, - tb), after two weeks of application (t2w) and after two further weeks of application (total 4 weeks, - t4w), both in the treated and in the not treated area.

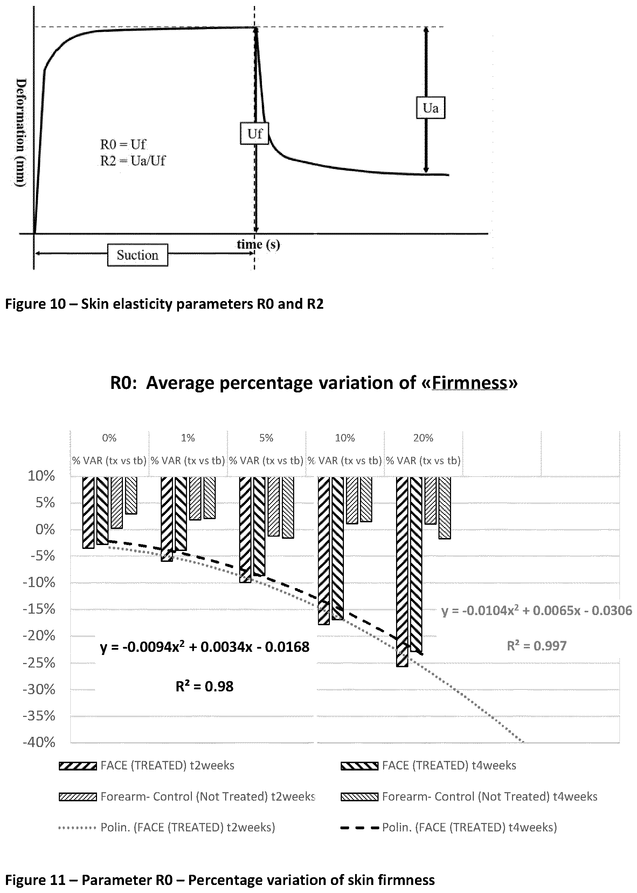

In this cutometry test, the percentages of increase/decrease of two of the most significant parameters of this kind of studies: R0 and R2 (Figure 10) were considered.

- The parameter of skin elasticity R0 (Figure 10) measures the maximal depth (in mm) of penetration of the skin into the probe when vacuum is applied (R0 = Uf); it indicates skin pliability and reflects the firmness/flexibility of the skin; it represents the passive behaviour in response to a force applied to the skin. Improving the compactness of skin normally implies a decrease of R0 (skin is less altered by the suction force).
- The parameter of skin elasticity R2 measures the ability of the skin to recover its initial state after that vacuum is stopped; it reflects the total skin elasticity - recovery/deformation - and is calculated according to the following formula:

$$R2 = Ua / Uf,$$

where (Figure 10):

Uf is the maximal distension of the skin at the end of the vacuum application period (i.e., Uf = R0)
Ua is the total recovery of the skin toward its original position after 1 second after stopping vacuum application (also defined as "gross elasticity recovery/deformation"). Its value is expressed as percentage.

Therefore, the parameter R2 reflects the elastic properties of the skin: it represents the proportion between the width of the deformation after suction and the ability to recover the initial position (relaxation/suction). The higher the recovery portion (time-delayed recovery), the better the elastic properties of skin.

An improvement of skin elasticity normally shows an increase of the value of R2.

The maximal penetration depth (mm) of the skin into the probe when vacuum is applied is defined R0 (= Uf).

The ability of the skin to recover its initial state after that vacuum is stopped is defined R2: Ua/Uf, wherein Uf is the maximal distension of the skin in the probe at the end of the suction period (Uf=R0) and Ua is the total recovery of the skin to its initial state after a second at a normal pressure.

**RESULTS**

The average values of R0 (Figure 11) of face skin treated with the vesicular concentrate at all the tested concentrations (20%, 10%, 5% 1%) were significantly lowered after 2 and 4 weeks of twice a day application, with respect to basal values (p<0.05).

An excellent, practically linear, dose-response correlation was found by comparing the variation of R0 for the incremental percentages of vesicular concentrate contained in the tested face creams in both series of data (2 weeks and 4 weeks application). [Again, the data correlate best with a quadratic distribution ($R^2$=0.9975 and $R^2$=0.987), but practically very near to a linear relationship because the coefficient of the quadratic term is very small and therefore negligible in both the correlations].

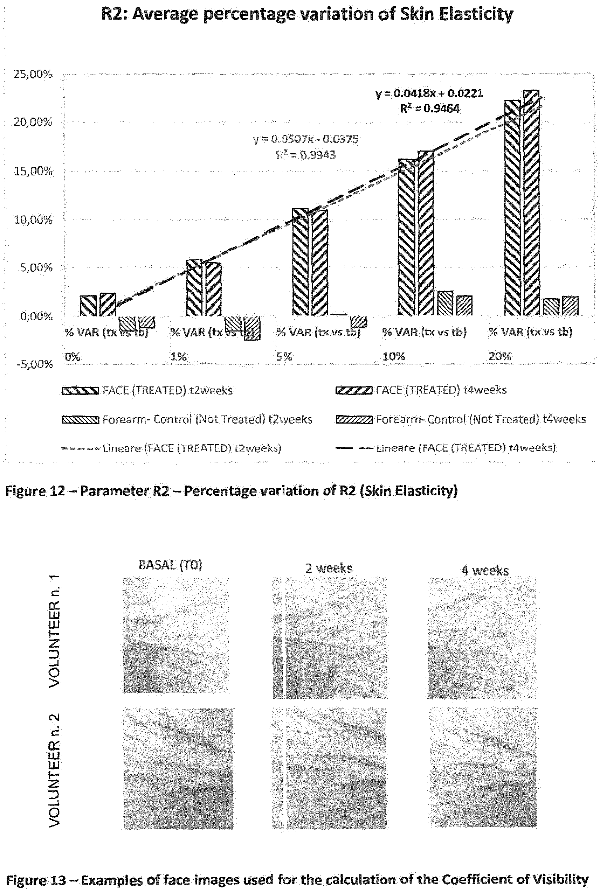

The average values of R2 (Figure 12) of skin in the face areas treated with 20%, 10% 5% and 1% vesicular concentrate were significantly increased after 2 and 4 weeks of a double daily application, with respect to the initial basal value (p<0.05).

In this case, too, an excellent linear dose-response correlation was found by comparing the variation percentage of R2 for the incremental quantities of vesicular concentrate in the tested formulation in the two series of data (2 weeks: $R^2$=0.9943; 4 weeks: $R^2$=0.9464).

Instead, no statistically significant variation for both R0 and R2 was found in the control area (not treated forearm).

Both for the parameter R0 and for the parameter R2 no significant variation was found (decrease and increase, respectively) of the value at 4 weeks of use of the product with respect to what was observed after the first two weeks, at all the tested quantities of vesicular concentrate.

Once again, like in the study on hydration, these results reflect the immediate effect of the vesicular concentrate on the skin: a high efficacy in improving the elasticity of the skin is reached in a relatively short application time (2 weeks).

The graph of Figure 11 shows the average percentage variation of the parameter R0 (compactness) after 2 and 4 consecutive weeks of double daily application of the face creams under evaluation (t2weeks, t4weeks) compared with the initial values before treatment (baseline, tb) in the treated areas (face) and the analogous values at the same measurement times for the not treated areas (forearm). The quadratic correlation curves for both series (2w and 4w) and the relative equations are shown, too.

The histogram of Figure 12 shows the average percentage of variation of the parameter R2 (skin elasticity) in the treated face areas after 2 and 4 consecutive weeks of daily application (twice a day) of the test products (t2weeks, t4weeks), with respect to the basal values before treatment (tb) compared with the analogous values for the not treated control area (forearm). The linear correlation curves for both series (2w and 4w) and the relative equations are shown, too.

- So, it was shown that the vesicular concentrate is effective in linearly improving the skin firmness/pliability (parameter R0) and the elastic properties (parameter R2) of the treated face skin.
- For both parameters R0 and R2 an excellent linear dose-response correlation was found.
- A high efficacy in the improvement of the elastic properties of the skin was obtained after just two weeks of use of the product, therefore a short term effect was verified, i.e. with results visible in a short period.

### 4.1.3 Wrinkles: Coefficient of wrinkle visibility (through image analysis).

The surface of the healthy skin shows a fine web of approximately rectilinear grooves, known as "skin microrelief" or "skin texture". This texture is made of parallel grooves of variable depth, intersecting each other to form rectangles, squares, lozenges, trapezoids and triangles.

The first consequence of this complex web of lines is an increase of 10% - 50% in the effective superficial area of the human body, with respect to the apparent area. This promotes exchanges between epidermis and environment, improving respiration, heat dispersion, and draining of sweat and sebum on long distances, so as to preserve an optimal hydration value.

Moreover, this texture contributes to skin extensibility.

The appearance of skin microrelief changes with increasing age: the highly regular scheme of the grooves in healthy, young skin is progressively replaced by a more disorganized structure, characterized by a decrease in the density of lines and an increase in their depth.

The efficacy of the vesicular concentrate in restoring the normal features of microrelief, in attenuating the appearance of thin lines and in improving skin smoothness and the overall appearance of skin was tested.

The chosen method is based on the visibility of wrinkles in face pictures (high quality standardized photographs taken in parallel polarized light conditions, with front and/or profile face +/- 45°- HeadScan Dynamics, OrionTechnoLab, France) (Figure 13). A dedicated software ensures the acquisition of images in reproducible lighting and shooting conditions. The photographic system employed was made of a Nikon camera provided with a 24 Mpx sensor. The continuous spectrum lighting had a CRI of 96 for a colour temperature of 4500 K°. The system was also equipped with an internal 24 colour chart allowing real time control of colorimetry and proper exposition of images.

The potential efficacy of basal formulas of face creams containing increasing percentages ofvesicular concentrate (0%: placebo; 1%; 5%; 10%; 20%) in improving (reducing) the visibility of wrinkles was evaluated, testing them on a panel of 50 volunteers (Caucasian women, age 44 - 66) - 10 volunteers for each formula. Each group applied the assigned formula twice a day for 4 consecutive weeks, while three measurements (image acquisitions) were performed: one before the application of the product (tb), an intermediate after 2 weeks of application (t2w), and a final at the end of 4 weeks of use (t4w). The area of the face chosen for taking the measurements was the lateral external periocular area (Figure 13).

Different parameters were calculated from these images obtained in the same face area for each volunteer at the different treatment time, using a software dedicated for the analysis of digital images (FrameScanV3, OrionTechnoLab, France), allowing to evaluate the intensity of face wrinkles by measuring the contrasts in images (number of lines, dimensions of wrinkles, contrast of the wrinkle with its surrounding).

Then an Index of Wrinkle Visibility was calculated - having a strong correlation with their clinical perception:

**Coefficient** of visibility = (Wrinkle Occupancy Rate) * (Contrast wrinkle/skin) * 100

More than a measurement of wrinkle depth (like the one obtainable with a 3D analysis), this coefficient is a numeric evaluation of wrinkle appearance and of their visual perception.

In this experiment, a reduction in the appearance of wrinkles corresponds to a decrease of the value of the visibility coefficient, as shown in Figure 13, which shows photographs of the same face area for the volunteers n. 1 and n. 2, taken at tb (before treatment) and after 2 and 4 weeks of use of the product. The software for image analysis allows to calculate the differences between them by measuring their contrast. A qualitative evaluation of wrinkle reduction can already be assessed with visual (clinical) observation of images at different times.

**RESULTS**

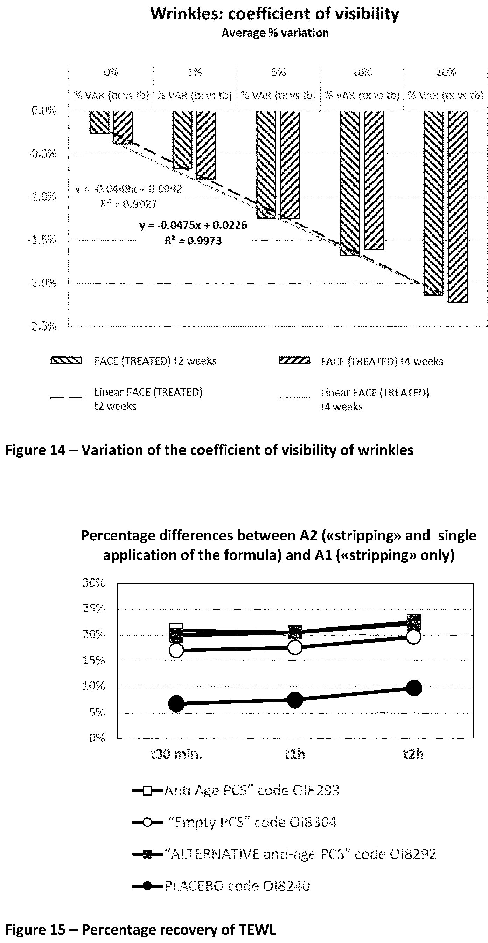

The results of this study are summarized in Figure 14 as graphical representation. The graph shows average percentage variations of the coefficient of visibility of wrinkles in the treated face areas, after 2 and 4 consecutive weeks of application of products (twice a day) with respect to basal value (tb).

There are also provided the linear correlation curves for the two series of values at 2 and 4 weeks, with their respective equations, too.

The coefficient of wrinkle visibility, in the face areas treated with all the formulations containing vesicular concentrate, was significantly decreased after 2 weeks of daily application of the products under test, with respect to the initial basal value. Therefore, the vesicular concentrate is effective in significantly reducing the visibility of wrinkles at all the tested concentrations, and such effect is reached already in a relatively short use period (2 weeks).

This reduction of wrinkle visibility linearly correlates very well with the quantity of vesicular concentrate applied on the skin for both series of data (after 2 weeks: $R^2 = 0.9973$; after 4 weeks: $R^2 = 0.9927$).

In the case of this clinical study, too, the coefficient of wrinkle visibility does not show a further decrease after two further weeks of application of the products (the differences between the values at 2 and 4 weeks for each concentration of vesicular concentrate are not statistically significant). Once more, this result testifies the "rapid" effect connected to the "physical" benefits provided to skin by the vesicular concentrate.

- The vesicular concentrate system is effective in significantly reducing the visibility of wrinkles at all tested concentrations.
- The reduction in the visibility of wrinkles linearly correlates with the quantity of vesicular concentrate applied on the skin.
- This beneficial effect is reached in the relatively short time of 2 weeks, once more accounting for the "rapid efficacy" correlated to the beneficial physical interaction of the vesicular concentrate with skin.

These results on wrinkle visibility were confirmed by the second study, described below in this document, that had such evaluation among the considered endpoints.

Overall, for all the three considered endpoints in the above-described studies (hydration, skin compactness, wrinkle visibility):

- All the tested quantities of vesicular concentrate were effective, with an excellent linear dose-response correlation. No plateau was detected. No dose threshold is needed for efficacy.

The specific endpoints selected for these studies - all direct or indirect indicators of a skin in a good state of health - are linked to the capacity of the vesicular concentrate to perfectly blend to skin meant as a barrier - in that the concentrate is biomimetic to skin - potentiating the protection capacity of skin and widening its intrinsic functionality, so inducing a rapid renewing/rejuvenating activity and with results easy perceptible in terms of improvement of skin tone and wrinkle reduction.

Overall, the vesicular concentrate is a flexible instrument with a wide use: added to cosmetic formulas can bring - proportionally to any employed quantity - its beneficial effects to skin barrier, allowing a free use of it by the cosmetic formulator in a wide range of cosmetic applications. The benefits of a rich cream can be obtained even with a "light" serum containing vesicular concentrate.

**Efficacy in enhancing skin barrier**

In this second clinical study, systems of vesicular concentrate formulated differently in the quantities of their constituting components were explored, so as to investigate the flexibility of the vesicular concentrate, to be understood more as a platform than as a complex cosmetic active ingredient made of a specific formula. For this reason, two endpoints (hydration and wrinkle visibility) already seen in the above study were evaluated again, to which a further specific endpoint was added in order to evaluate the protection action and reparation of skin barrier.

The formulas tested in this study were identical, and differed only for the systems of vesicular concentrate inside them - systems formulated differently according to the quantities of their constituent components. Therefore, four formulas of a basal face cream were prepared, three containing each 10% of a different vesicular concentrate:

- Anti Age PCS OI8293: Example 2
- Empty PCS OI8304: Example 1
- ALTERNATIVE Anti-Age PCS OI8292: Example 2 bis
- a fourth without vesicular concentrate as placebo.

The clinical tests were conducted on 40 volunteers (age 40-65, women) subdivided into 4 groups of 10; one of the four formulas was assigned to each group.

For the TEWL endpoint the study was performed as indicated below.

For Hydration and Coefficient of Visibility, the product was applied on the face the first time in the clinical centre, under controlled conditions.

Successively, the volunteers continued the use of product by applying it two times a day (morning and evening) for a period of 2 consecutive weeks (14 days), according to directions. Then they suspended the use of the test product and of any other cosmetic product on their face for 7 days.

The instrumental measurements of hydration and coefficient of visibility were performed on the face of the volunteers treated with the assigned product (test area) and on a not treated area of the forearm (control area) at the following times: just before product application (tb), after 2 hours since the first application of the product (t2h), after two weeks (14 days) of daily application (twice a day) of the product (t2w), 7 days after suspending the application of the product and of any other cosmetic product (t3w).

Given the influence that temperature and relative humidity have on the skin, the volunteers were kept for at least 20 minutes under controlled conditions (range: $22 \pm 2°C$ and $50 \pm 5\%$ respectively) before measurement.

### 5.1.1 TEWL (Trans Epidermal Water Loss)
### 5.1.2 Hydration
### 5.1.3 Wrinkle visibility

### 5.1.1 Enhancing effect on skin barrier

Protection against environmental stress - immediate effect measured through TEWL (Trans-Epidermal Water Loss) after stripping.

TEWL is a measure of the integrity of the "barrier function" of skin. By inducing an initial light damage to skin under controlled condition (*stripping* of the skin for 15 consecutive times) an increase in TEWL is determined.

The efficacy of the vesicular concentrate in protecting/repairing the so altered skin barrier was evaluated by comparing the recovery rate of TEWL in a short time (30, 60 e 120 minutes) of damaged skin and treated with vesicular concentrate with respect to damaged and not treated skin, and also by comparing with the behaviour of a placebo formula not containing vesicular concentrate.

### RESULTS

The results of this study are summarized in Figure 15 as graphic representation. The graph shows the recovery percentages of the TEWL value, after damaging (skin stripping) for the different formulas containing vesicular concentrate and for the placebo, with respect to damaged skin not treated with any product.

All the tested formulas allowed a rapid recovery of the skin barrier (TEWL reduction) in a significantly higher way with respect to the placebo formula (not containing vesicular concentrate) tested for comparison.

### 5.1.2 Increase in Hydration

Similarly to what was described for the first study, in this case, too, the hydrating efficacy of the tested formulas was tested through instrumental measures of skin hydration (Corneometer CM825 - Courage + Khazaka) after a single application (2h), after repeated daily applications for 14 days, and after further 7 days after suspending treatment.

### RESULTS

The data on skin hydration obtained from the experiment - statistically evaluated for their validity - are reported in the following table, wherein there are provided the percentage difference for each tested product at different measurement time with respect to basal value.

As can be easily observed, all the three cream formulas containing the vesicular concentrate were very effective in increasing immediately (value at 2 hours) the hydration value, which remained constant during the whole use period of the products. This increase is markedly higher than that reached by placebo, which has the same formula but does not contain vesicular concentrate.

The small differences detected for the not treated areas (forearm) were not statistically significant.

One week after suspending the application, hydration goes essentially back to initial values.

|  | FACE ΔX | | | Forearm not treated control | | |
|---|---|---|---|---|---|---|
|  | t2hours | t2w | t3w after 1w of suspension | t2hours | t2w | 3w after 1 w of suspension |
| "Anti Age PCS" code 018293 | 32.20% | 33.09% | 2.70% | 2.58% | 2.88% | 0.53% |
| "Empty PCS" code 018304 | 30.51% | 28.57% | 1.17% | 1.08% | 1.52% | 1.38% |
| "ALTERNATIVE anti-age PCS" code 018292 | 32.73% | 32.86% | 2.46% | -0.26% | 0.43% | -0.50% |
| PLACEBO code 018240 | 6.67% | 6.44% | 0.33% | 1.24% | 2.49% | 0.24% |

By comparing the values of increment of hydration at the different measurement times reached by formula OI8304 in this experiment (+ 30.51% at 2 hours; + 28.57 at 14 days) and the same formula tested in the first experiment (vesicular concentrate 10%: + 24.59% at 6 hours; +23.91% at 14 days) a good overlapping of results can be observed, considering that they were obtained on two small groups of volunteers (10 each) different from each other and in different times.

### 5.1.3 Improvement in the appearance of face skin

### Reduction in the Coefficient of Visibility of wrinkles

As explained for the above first clinical study, the objective here is evaluating efficacy in restoring the normal features of skin microrelief, attenuating the appearance of thin wrinkles and in improving the smoothness and the general appearance of skin.

Summarizing what is more amply described above, this method is based on the measurement of wrinkles visibility in photograph images of face, obtained with a system allowing to acquire standardized photographs in polarized parallel light, with front and profile face (+/- 45° - HeadScan Dynamics, OrionTechnoLab, France). A dedicated software ensures the acquisition under reproducible lighting and shooting conditions.

From these images an Index of "Visibility of Wrinkles" is calculated, which is very correlated to the clinical perception of wrinkles.

**Coefficient of Visibility = (Occupancy rate of wrinkles) * (Contrast wrinkle/skin) * 100**

### RESULTS

The results of this part of the study are summarized in the following table.

|  | TREATED FACE | | | % variation | % variation |
|---|---|---|---|---|---|
|  | tb | t2w | t3w (7 days after the end of formula application) | t2w *vs* tb | t3w (7 days after the end of formula application) *vs* tb |
| Anti Age PCS" OI8293 | 1.145 | 0.931 | 1.106 | **-19.37%** | **-4.70%** |
| "Empty PCS" OI8304 | 1.458 | 1.170 | 1.408 | **-18.32%** | **-3.69%** |

(continued)

| | TREATED FACE | | | % variation | % variation |
|---|---|---|---|---|---|
| | tb | t2w | t3w (7 days after the end of formula application) | t2w *vs* tb | t3w (7 days after the end of formula application) *vs* tb |
| "ALTERNATIVE Anti-Age PCS" 018292 | 1.308 | 1.058 | 1.223 | **-19.07%** | **-4.56%** |
| PLACEBO OI8240 | 1.553 | 1.512 | 1.544 | **-2.63%** | **-0.62%** |

In this case, too, it can be noted that the tested vesicular concentrates behave nearly in the same way: all of them allow a significant reduction of the visibility of wrinkles, while the placebo is not very effective.

Suspending the use of products, the coefficient of visibility goes back nearly to the basal value, similarly to what was observed for the same parameter in the first study.

Overall, for the three endpoints considered in the study, no significant difference emerged between the different systems of tested vesicular concentrate, confirming that the vesicular concentrates can be modified in their composition in order to adapt to the different cosmetic applications, while maintaining their high efficacy.

Figure 16 shows what occurs on the skin after the application of a cream containing the vesicular concentrate according to the present invention. During the application, vesicles are distanced thanks to the presence of water in the cream (first scheme on the right). After the natural evaporation of water, vesicles flatten out forming a protective film (second scheme on the right). The two photographs CRYO-TEM and TEM on the left show their effect on the skin.

Figure 17 shows a comparison between micro vesicles on the left and nano vesicles on the right. The evaporation of water being constant, the vesicles of micrometric dimensions (0.6-1.0 $\mu$m) like those according to the present invention form a much more effective barrier in comparison to that made of vesicles of nanometric dimensions (< 100 nm of diameter).

Evidently, the barrier action performed by the vesicles adds up to the action performed by the active ingredients that can be vehiculated by the vesicles like panthenol, avena sativa (oat) kernel protein, ectoin, 3-o-ethyl-ascorbic acid, niacinamide, which require a longer time (2-4 weeks) to deploy their effect.

**Example 1 - Formulation of a vesicular concentrate**

| PHASE | INGREDIENTS | % WEIGHT |
|---|---|---|
| PHASE A | | |
| | GLYCEROL | 36 |
| (a) | HYDROGENATED PHOSPHATIDYLCHOLINE | 10 |
| (a) | LECITHIN | 2 |
| (d) | SODIUM BETA-SITOSTERYL SULFATE | 0.20 |
| (c) | PHYTOSTERYL ISOSTEARATE | 0.60 |
| (c) | BETA-SITOSTEROL | 2 |
| (e) | CERAMIDE NP | 2 |
| (b) | PHYTOSPHINGOSINE | 2 |
| | SQUALANE | 10 |
| | TOCOPHERYL ACETATE | 0.1 |
| | CAPRYLYL GLYCOL | 1.1 |
| PHASE B | | |
| | WATER | 30 |
| | PANTHENOL | 4 |

(continued)

| PHASE B | | |
|---|---|---|
| TOTAL | | 100.00 |

**Example 2 - Formulation of a vesicular concentrate charged with lipophilic and hydrophilic active ingredients**

| PHASE | INGREDIENTS | % WEIGHT |
|---|---|---|
| PHASE A | | |
| | GLYCEROL | 36 |
| | HYDROGENATED PHOSPHATIDYLCHOLINE | 10 |
| | LECITHIN | 1 |
| | LYSOLECITHIN | 1 |
| | SODIUM BETA-SITOSTERYL SULFATE | 0.20 |
| | PHYTOSTERYL ISOSTEARATE | 0.60 |
| | BETA-SITOSTEROL | 2 |
| | CERAMIDE NP | 2 |
| | PHYTOSPHINGOSINE | 2 |
| | SQUALANE | 10 |
| | TOCOPHERYL ACETATE | 0.1 |
| | TOCOPHERYL NICOTINATE | 1.5 |
| | CAPRYLYL GLYCOL | 1.1 |
| PHASE B | | |
| | WATER | 26 |
| | AVENA SATIVA (OAT) KERNEL PROTEIN | 1 |
| | ECTOIN | 1.5 |
| | PANTHENOL | 4 |
| TOTAL | | 100.00 |

**Example 2 bis - Formulation of a vesicular concentrate charged with lipophilic and hydrophilic active ingredients**

| PHASE | INGREDIENTS | % WEIGHT |
|---|---|---|
| PHASE A | | |
| | GLYCEROL | 36 |
| | HYDROGENATED PHOSPHATIDYLCHOLINE | 5 |
| | LECITHIN | 7 |
| | SODIUM BETA-SITOSTERYL SULFATE | 0.20 |
| | PHYTOSTERYL ISOSTEARATE | 0.60 |
| | BETA-SITOSTEROL | 2 |

(continued)

| PHASE | INGREDIENTS | % WEIGHT |
|---|---|---|
| PHASE A | | |
| | CERAMIDE NP | 2 |
| | PHYTOSPHINGOSINE | 2 |
| | SQUALANE | 10 |
| | TOCOPHERYL ACETATE | 0.1 |
| | TOCOPHERYL NICOTINATE | 1.5 |
| | CAPRYLYL GLYCOL | 1.1 |
| PHASE B | | |
| | WATER | 27 |
| | ECTOIN | 1.5 |
| | PANTHENOL | 4 |
| TOTAL | | 100.00 |

**Example 3 - Formulation of a vesicular concentrate charged with lipophilic and hydrophilic active ingredients**

| PHASE | INGREDIENTS | % WEIGHT |
|---|---|---|
| PHASE A | | |
| | GLYCERIN | 36 |
| | HYDROGENATED PHOSPHATIDYLCHOLINE | 10 |
| | LECITHIN | 2 |
| | SODIUM BETA-SITOSTERYL SULFATE | 0.20 |
| | PHYTOSTERYL ISOSTEARATE | 0.60 |
| | BETA-SITOSTEROL | 2 |
| | CERAMIDE NP | 2 |
| | PHYTOSPHINGOSINE | 2 |
| | SQUALANE | 10 |
| | TOCOPHERYL ACETATE | 0.1 |
| | UNDECYLENOYL PHENYLALANINE | 0.75 |
| | HYDROXYPINACOLONE RETINOATE | 0.75 |
| | CAPRYLYL GLYCOL | 1.1 |
| PHASE B | | |
| | WATER | 27 |
| | 3-O-ETHYL-ASCORBIC ACID | 0.75 |
| | NIACINAMIDE | 0.75 |
| | PANTHENOL | 4 |
| TOTAL | | 100.00 |

**Example 3 bis - Formulation of a vesicular concentrate charged with lipophilic and hydrophilic active ingredients with addition of a fatty acid**

| PHASE | INGREDIENT | % WEIGHT |
|---|---|---|
| PHASE A | | |
| | GLYCERIN | 36 |
| | HYDROGENATED PHOSPHATIDYLCHOLINE | 10 |
| | LECITHIN | 2 |
| | LYSOLECITHIN | 1 |
| | SODIUM BETA-SITOSTERYL SULFATE | 0.20 |
| | PHYTOSTERYL ISOSTEARATE | 0.60 |
| | BETA-SITOSTEROL | 2 |
| | CERAMIDE NP | 2 |
| | PHYTOSPHINGOSINE | 2 |
| | SQUALANE | 9 |
| | LINOLEIC ACID | 1 |
| | TOCOPHERYL ACETATE | 0.1 |
| | TOCOPHERYL NICOTINATE | 1.5 |
| | CAPRYLYL GLYCOL | 1.1 |
| PHASE B | | |
| | WATER | 26 |
| | AVENA SATIVA (OAT) KERNEL PROTEIN | 1 |
| | ECTOIN | 1.5 |
| | PANTHENOL | 4 |
| TOTAL | | 100.00 |

**Example 3 ter - Formulation of a vesicular concentrate with a higher content of phospholipids**

| PHASE | INGREDIENTS | % WEIGHT |
|---|---|---|
| PHASE A | | |
| | GLYCERIN | 20 |
| | HYDROGENATED PHOSPHATIDYLCHOLINE | 16 |
| | LECITHIN | 12 |
| | SODIUM BETA-SITOSTERYL SULFATE | 0.20 |
| | PHYTOSTERYL ISOSTEARATE | 0.60 |
| | BETA-SITOSTEROL | 2 |
| | CERAMIDE NP | 2 |
| | PHYTOSPHINGOSINE | 2 |
| | SQUALANE | 10 |

(continued)

| PHASE | INGREDIENTS | % WEIGHT |
|---|---|---|
| PHASE A | | |
| | TOCOPHERYL ACETATE | 0.1 |
| | CAPRYLYL GLYCOL | 1.1 |
| PHASE B | | |
| | WATER | 30 |
| | PANTHENOL | 4 |
| TOTAL | | 100.00 |

The content of (a) to (e) ingredients in the different examples 1-3 ter is always ranging 15-40% as indicated in claim 1.

**Example 4 - Formulation of a face cream with a vesicular concentrate at 10%**

| Ingredients | % Weight |
|---|---|
| WATER | 62.85 |
| CELLULOSE | 15.00 |
| XYLITOL | 2.20 |
| BETAINE | 0.60 |
| SODIUM HYALURONATE | 0.05 |
| CARBOMER | 0.70 |
| PENTYLENE GLYCOL | 2.20 |
| SORBITAN CAPRYLATE | 0.25 |
| CAPRYLIC/CAPRIC TRIGLYCERIDE | 2.00 |
| SQUALANE | 1.50 |
| BEHENYL ALCOHOL | 2.00 |
| ARGININE | 0.65 |
| VESICULAR CONCENTRATE ACCORDING TO EXAMPLE 1, 2, 3 | 10.00 |
| TOTAL | 100.00 |

**Example 5 - Formulation of a face serum with a vesicular concentrate at 5%**

| Ingredients | % Weight |
|---|---|
| WATER | 91.00 |
| ERYTHRITOL | 3.00 |
| TAMARINDUS INDICA SEED GUM | 0.60 |
| HYDROXYACETOPHENONE | 0.15 |
| PHENOXYETHANOL | 0.25 |
| VESICULAR CONCENTRATE ACCORDING TO EXAMPLE 1, 2, 3 | 5.00 |

(continued)

| Ingredients | % Weight |
| --- | --- |
| TOTAL | 100.00 |

**Example 6 - Formulation of a face serum with a vesicular concentrate at 20%**

| Ingredients | % Weight |
| --- | --- |
| WATER | 72.00 |
| XYLITOL | 1.50 |
| XANTHAM GUM | 0.40 |
| POLYQUATERNIUM-37 | 0.30 |
| SODIUM HYALURONATE | 0.05 |
| BETAINE | 1.45 |
| PENTYLENE GLYCOL | 2.00 |
| SORBITAN CAPRYLATE | 0.20 |
| CAPRYLIC/CAPRIC TRIGLYCERIDE | 1.10 |
| SQUALANE | 1.00 |
| VESICULAR CONCENTRATE ACCORDING TO EXAMPLE 1, 2, 3 | 20.00 |
| TOTAL | 100.00 |

**Example 7 - Formulation of a body cream with vesicular concentrate at 24%**

| Ingredients | % Weight |
| --- | --- |
| WATER | 42.50 |
| CELLULOSE | 14.00 |
| XYLITOL | 2.00 |
| BETAINE | 0.60 |
| CYAMOPSIS TETRAGONOLOBA GUAR GUM | 0.60 |
| PENTYLENE GLYCOL | 2.00 |
| SORBITAN CAPRYLATE | 0.30 |
| CAPRYLIC/CAPRIC TRIGLYCERIDE | 3.50 |
| SQUALANE | 3.00 |
| BEHENYL ALCOHOL | 7.00 |
| DIMETHICONE | 0.50 |
| VESICULAR CONCENTRATE ACCORDING TO EXAMPLE 1, 2, 3 | 24.00 |
| TOTAL | 100.00 |

**Example 8 - Formulation of a powder product**

| PHASE | INGREDIENTS | % WEIGHT |
|---|---|---|
| PHASE A | | |
| | WATER | 10 |
| | VESICULAR CONCENTRATE ACCORDING TO EXAMPLES 1, 2 OR 3 | 10 |
| PHASE B | | |
| | MICA | 62 |
| | IRON OXIDES | 10 |
| | SILICA | 5 |
| | CELLULOSE | 1 |
| | CAPRYLYL GLYCOL | 2 |
| TOTAL | | 100.00 |

The example 8 provides the mixing of Phase A with Phase B through a powder mixer and the drying of the powder treated at 50°C in a ventilated oven up to complete drying, followed by meshing through sieves mesh 120 and compacting in godet.

## Claims

1. Cosmetic product having a composition comprising a vesicular concentrate consisting of a water solution of lipidic vesicles, and any further cosmetically acceptable ingredients, **characterized in that** said lipid vesicles are multilamellar vesicles of dimensions ranging 0.6-1.0 microns with walls formed by the following ingredients:

   (a) phospholipids
   (b) phytosphingosine
   (c) phytosterol
   (d) beta-phytosteryl sulfate and optionally other derivates of phytosterol
   (e) ceramides and/or pseudoceramides

   wherein the weight content of the (a) to (e) ingredients ranges 15-40% of the vesicular concentrate composition.

2. Cosmetic product according to claim 1, wherein said vesicular concentrate forms a film on skin.

3. Cosmetic product according to claim 1, wherein all the ingredients are vegetal ingredients or are of vegetal origin.

4. Cosmetic product according to claim 1 or 2, wherein said vesicular concentrate further contains a lysophospholipid as ingredient (f).

5. Cosmetic product according to any preceding claim, wherein said vesicular concentrate further contains an ester of phytosterol as ingredient (g).

6. Cosmetic product according to any preceding claim, wherein said vesicular concentrate further contains one or a mixture of fatty acids, particularly oleic acid, palmitic acid, fatty acids of coconut oil, as ingredient (h).

7. Cosmetic product according to any preceding claim, wherein said vesicular concentrate further contains one or a mixture of humectants, in particular glycerol, xylitol, sorbitol, isosorbide, butylene glycol, as ingredient (i).

8. Cosmetic product according to any preceding claim, wherein said vesicular concentrate further contains one or a mixture of emollients, in particular phytosqualane, phytosqualene, emisqualane, caprylyl/capric triglyceride, cetyl palmitate, cetearyl ethylhexanoate octyldodecanol, dimethicone, as ingredient (l).

9. Cosmetic product according to any preceding claim, which further contains one or a mixture of preservatives, in particular sorbitan caprylate, 1,2-hexylene glycol, hydroxy acetophenone, as ingredient (m).

10. Cosmetic product according to any preceding claim, wherein said vesicular concentrate further contains one or a mixture of lipophilic active molecules, in particular acetate of tocopheryl, nicotinate of tocopheryl, retinol, tocotrienol, ethyl ferulate, ethyl linoleate, as ingredient (n).

11. Cosmetic product according to any preceding claim, wherein said vesicular concentrate further contains one or a mixture of hydrophilic active molecules, in particular ectoin, d-panthenol, nmf factor, glucosyl ascorbate, sodium ascorbyl phosphate, myo-inositol, acetyl glucosamine, alanyl glutamine, acetylcysteine, as ingredient (o).

12. Cosmetic product according to any preceding claim, which further contains one or a mixture of thickening agents, in particular xanthan gum, cellulose gum, hyaluronan, glucan, reticulated polymers of sodium acrylate, poliquaternium-37, carrageenan, alginic derivatives, guar gum derivatives, as ingredient (p).

13. Cosmetic product according to any preceding claim, wherein said vesicular concentrate further contains one or a mixture of film-forming molecules, in particular corn protein, oat protein, rice protein, hydrolysed rice protein, as ingredient (q).

14. Cosmetic product according to any of claims 1-13, comprising from 0.1% to 60% by weight of said vesicular concentrate.

15. Method for obtaining a cosmetic product according to claim 1 or 14, comprising:

    • preparing vegetal or of vegetal origin biomimetic vesicles with dimensions ranging 0.6-1.0 microns with walls comprising a lipid phase with the ingredients of any of claims 1-13, with the following sub-steps:

        ◦ preparing a composition containing a vesicular concentrate through melting of the walls of said vesicles up to the liquid-crystalline melt state so as to obtain a homogeneous monophasic system;
        ◦ introducing a water phase into said vesicular concentrate composition at a temperature higher than the solidification temperature of the lipid phase of the vesicles;
        ◦ cooling of the vesicular concentrate composition below the solidification temperature of the lipid phase of the vesicles until obtaining a material with pasty texture having pseudoplastic fluid or viscoelastic solid rheologic characteristics with size distribution of the vesicles comprised between 0.6 and 1.0 micron;

    • combining the obtained vesicular concentrate composition with pasty texture with water and other cosmetic ingredients up to obtaining a finished cosmetic product containing a desired weight percentage of said vesicular concentrate.

16. Method for preparing a cosmetic product according to claim 15, wherein said vesicular concentrate is obtained through the following steps:

    • inserting the ingredients of the vesicle walls (PHASE A) one at a time into a jacketed mixer provided with turbine (rotor-stator), mechanical stirring and wiper;
    • mechanical stirring of the mix at room temperature up to obtaining a smooth, homogeneous dispersion of the components;
    • gradual heating of PHASE A by increments of 10°C over a period of 1 hour up to the temperature of 80-100°C in order to allow the melting of solid components obtaining a translucent dispersion with pasty texture and slightly yellowish;
    • heating of PHASE A at 60°C continuing the mechanical stirring;
    • gradual addition of the components to be added inside said vesicles (PHASE B) to the mixer, keeping the temperature constant;
    • once the addition of the PHASE B is completed, stirring of the composition for 30 minutes;
    • turbulent stirring of the mix actuating said turbine and gradually reducing the temperature up to room temperature, obtaining a concentrate with a white/yellowish paste appearance.

17. Method according to claim 15, wherein said other cosmetic ingredients consists of cosmetic powders, and there is provided a final drying step for removing water and the obtainment of a powder cosmetic product containing said vesicular concentrate composition and cosmetic powders.

**Patentansprüche**

1. Kosmetisches Produkt mit einer Zusammensetzung, die ein vesikuläres Konzentrat umfasst, das aus einer wässrigen Lösung von Lipidvesikeln besteht, und irgendwelchen weiteren kosmetisch akzeptablen Inhaltsstoffen, **dadurch gekennzeichnet, dass** die Lipidvesikel multilamellare Vesikel mit Abmessungen im Bereich von 0,6 - 1,0 Mikrometer sind, deren Wände aus den folgenden Inhaltsstoffen gebildet sind:

   (a) Phospholipide
   (b) Phytosphingosin
   (c) Phytosterol
   (d) Beta-Phytosterylsulfat und optional andere Derivate von Phytosterol
   (e) Ceramide und/oder Pseudoceramide

   wobei der Gewichtsanteil der Bestandteile (a) bis (e) 15 - 40 % der vesikulären Konzentratzusammensetzung beträgt.

2. Kosmetisches Produkt gemäß Anspruch 1, wobei das vesikuläre Konzentrat einen Film auf Haut bildet.

3. Kosmetisches Produkt gemäß Anspruch 1, wobei alle Inhaltsstoffe pflanzliche Inhaltsstoffe sind oder pflanzlichen Ursprungs sind.

4. Kosmetisches Produkt gemäß Anspruch 1 oder 2, wobei das vesikuläre Konzentrat ferner ein Lysophospholipid als Inhaltsstoff (f) enthält.

5. Kosmetisches Produkt gemäß irgendeinem vorstehenden Anspruch, wobei das vesikuläre Konzentrat ferner einen Phytosterolester als Bestandteil (g) enthält.

6. Kosmetisches Produkt gemäß irgendeinem vorstehenden Anspruch, wobei das vesikuläre Konzentrat ferner eine oder eine Mischung von Fettsäuren, insbesondere Ölsäure, Palmitinsäure, Fettsäuren aus Kokosnussöl, als Bestandteil (h) enthält.

7. Kosmetisches Produkt gemäß irgendeinem vorstehenden Anspruch, wobei das vesikuläre Konzentrat ferner eines oder eine Mischung von Feuchthaltemittel, insbesondere Glycerin, Xylitol, Sorbit, Isosorbid, Butylenglykol, als Inhaltsstoff (i) enthält.

8. Kosmetisches Produkt gemäß irgendeinem vorstehenden Anspruch, wobei das vesikuläre Konzentrat ferner einen oder eine Mischung von Emollientien, insbesondere Phytosqualan, Phytosqualen, Emisqualan, Caprylyl-/Capric-Triglycerid, Cetylpalmitat, Cetearylethylhexanoat, Octyldodecanol, Dimethicon, als Inhaltsstoff (1) enthält.

9. Kosmetisches Produkt gemäß irgendeinem vorstehenden Anspruch, welches ferner einen oder eine Mischung von Konservierungsstoffen, insbesondere Sorbitancaprylat, 1,2-Hexylenglykol, Hydroxyacetophenon, als Bestandteil (m) enthält.

10. Kosmetisches Produkt gemäß irgendeinem vorstehenden Anspruch, wobei das vesikuläre Konzentrat ferner ein oder eine Mischung von lipophilen Wirkmolekülen, insbesondere Tocopherylacetat, Tocopherylnicotinat, Retinol, Tocotrienol, Ethylferulat, Ethyllinoleat, als Inhaltsstoff (n) enthält.

11. Kosmetisches Produkt gemäß irgendeinem vorstehenden Anspruch, wobei das vesikuläre Konzentrat ferner einen oder eine Mischung von hydrophilen Wirkstoffen, insbesondere Ectoin, D-Panthenol, NMF-Faktor, Glucosylascorbat, Natriumascorbylphosphat, Myo-Inositol, Acetylglucosamin, Alanylglutamin, Acetylcystein, als Inhaltsstoff (o) enthält.

12. Kosmetisches Produkt gemäß irgendeinem vorstehenden Anspruch, welches ferner eines oder eine Mischung von Verdickungsmitteln, insbesondere Xanthangummi, Cellulosegummi, Hyaluronsäure, Glucan, vernetzte Polymere aus Natriumacrylat, Poliquaternium-37, Carrageen, Alginsäurederivate, Guarkernmehlderivate, als Inhaltsstoff (p) enthält.

13. Kosmetisches Produkt gemäß irgendeinem vorstehenden Anspruch, wobei das vesikuläre Konzentrat ferner ein oder eine Mischung von filmbildenden Molekülen, insbesondere Maisprotein, Haferprotein, Reisprotein, hydrolysiertes Reisprotein, als Inhaltsstoff (q) enthält.

14. Kosmetisches Produkt gemäß irgendeinem der Ansprüche 1 bis 13, das 0,1 bis 60 Gew.-% des vesikulären Konzentrats umfasst.

15. Verfahren, mit dem ein kosmetisches Produkt gemäß Anspruch 1 oder 14 erhalten wird, umfassend:

 • Herstellen von biomimetischen Vesikeln aus pflanzlichen Stoffen oder pflanzlichen Ursprungs mit Abmessungen im Bereich von 0,6 bis 1,0 Mikrometern, deren Wände eine Lipidphase mit den Bestandteilen irgendeines der Ansprüche 1 bis 13 umfassen, mit den folgenden Teilschritten:

  o Herstellen einer Zusammensetzung, die ein vesikuläres Konzentrat enthält, durch Schmelzen der Wände der Vesikel bis zum flüssigkristallinen Schmelzzustand, damit ein homogenes einphasiges System erhalten wird;
  o Einbringen einer Wasserphase in die vesikuläre Konzentratzusammensetzung bei einer Temperatur, die höher ist als die Erstarrungstemperatur der Lipidphase der Vesikel;
  o Abkühlen der vesikulären Konzentratzusammensetzung unter die Erstarrungstemperatur der Lipidphase der Vesikel, bis ein Material mit pastöser Textur erhalten wird, das pseudoplastische flüssige oder viskoelastische feste rheologische Eigenschaften mit einer Größenverteilung der Vesikel zwischen 0,6 und 1,0 Mikrometer umfasst;

 • Kombinieren der erhaltenen vesikulären Konzentratzusammensetzung mit pastöser Textur mit Wasser und anderen kosmetischen Inhaltsstoffen, bis ein fertiges kosmetisches Produkt erhalten wird, das einen gewünschten Gewichtsprozentsatz des vesikulären Konzentrats enthält.

16. Verfahren zur Herstellung eines kosmetischen Produkts gemäß Anspruch 15, wobei das vesikuläre Konzentrat durch die folgenden Schritte erhalten wird:

 • Einbringen der Bestandteile der Vesikelwände (PHASE A) nacheinander in einen Mantelmischer, der mit einer Turbine (Rotor-Stator), einem mechanischen Rührwerk und einem Abstreifer ausgestattet ist;
 • mechanisches Rühren der Mischung bei Raumtemperatur, bis eine glatte, homogene Dispersion der Komponenten erhalten wird;
 • allmähliches Erhitzen von PHASE A in Schritten von 10 °C über einen Zeitraum von 1 Stunde auf eine Temperatur von 80-100 °C, um das Schmelzen der festen Komponenten zu ermöglichen und eine durchscheinende Dispersion mit pastöser Textur und leicht gelblichem Farbton zu erhalten;
 • Erhitzen von PHASE A auf 60 °C unter zusammenhängendem mechanischem Rühren;
 • allmähliches Hinzufügen der in die Vesikel einzubringenden Komponenten (PHASE B) zum Mischer unter Beibehaltung einer konstanten Temperatur;
 • nach Beendigung der Zugabe von PHASE B 30-minütiges Rühren der Zusammensetzung;
 • turbulentes Rühren der Mischung unter Betätigung der Turbine und allmähliches Absenken der Temperatur auf Raumtemperatur, wodurch ein Konzentrat mit einem weiß/gelblichen pastösen Aussehen erhalten wird.

17. Verfahren gemäß Anspruch 15, wobei die anderen kosmetischen Inhaltsstoffe aus kosmetischen Pulvern bestehen und ein abschließender Trocknungsschritt vorgesehen ist, um Wasser zu entfernen und ein pulverförmiges Kosmetikprodukt zu erhalten, das die vesikuläre Konzentratzusammensetzung und die kosmetischen Pulver enthält.

**Revendications**

1. Produit cosmétique ayant une composition comprenant un concentré vésiculaire consistant en une solution aqueuse de vésicules lipidiques, et n'importe quels autres ingrédients acceptables en cosmétique, **caractérisé en ce que** lesdites vésicules lipidiques sont des vésicules multilamellaires ayant des dimensions allant de 0,6 à 1,0 micromètre avec des parois formées par les ingrédients suivants :

 (a) phospholipides
 (b) phytosphingosine
 (c) phytostérol
 (d) sulfate de bêta-phytostéryle et éventuellement autres dérivés de phytostérol
 (e) céramides et/ou pseudocéramides

dans lequel la teneur en poids en les ingrédients (a) à (e) va de 15 à 40 % de la composition de concentré vésiculaire.

2. Produit cosmétique selon la revendication 1, dans lequel ledit concentré vésiculaire forme un film sur la peau.

3. Produit cosmétique selon la revendication 1, dans lequel tous les ingrédients sont des ingrédients végétaux ou sont d'origine végétale.

4. Produit cosmétique selon la revendication 1 ou 2, dans lequel ledit concentré vésiculaire contient en outre un lysophospholipide à titre d'ingrédient (f).

5. Produit cosmétique selon l'une quelconque des revendications précédentes, dans lequel ledit concentré vésiculaire contient en outre un ester de phytostérol à titre d'ingrédient (g).

6. Produit cosmétique selon l'une quelconque des revendications précédentes, dans lequel ledit concentré vésiculaire contient en outre un acide gras ou un mélange d'acides gras, en particulier l'acide oléique, l'acide palmitique, les acides gras dérivés de l'huile de coprah, à titre d'ingrédient (h).

7. Produit cosmétique selon l'une quelconque des revendications précédentes, dans lequel ledit concentré vésiculaire contient en outre un humectant ou un mélange d'humectants, en particulier le glycérol, le xylitol, le sorbitol, l'isosorbide, le butylèneglycol, à titre d'ingrédient (i).

8. Produit cosmétique selon l'une quelconque des revendications précédentes, dans lequel ledit concentré vésiculaire contient en outre un émollient ou un mélange d'émollients, en particulier le phytosqualane, le phytosqualène, l'émisqualane, les triglycérides capryliques/capriques, le palmitate de cétyle, l'éthylhexanoate de cétéaryle, l'octyldodécanol, la diméthicone, à titre d'ingrédient (l).

9. Produit cosmétique selon l'une quelconque des revendications précédentes, qui contient en outre un conservateur ou un mélange de conservateurs, en particulier le caprylate de sorbitan, le 1,2-hexylèneglycol, l'hydroxyacétophénone, à titre d'ingrédient (m).

10. Produit cosmétique selon l'une quelconque des revendications précédentes, dans lequel ledit concentré vésiculaire contient en outre une molécule active lipophile ou un mélange de molécules actives lipophiles, en particulier l'acétate de tocophéryle, le nicotinate de tocophéryle, le rétinol, le tocotriénol, le férulate d'éthyle, le linoléate d'éthyle, à titre d'ingrédient (n).

11. Produit cosmétique selon l'une quelconque des revendications précédentes, dans lequel ledit concentré vésiculaire contient en outre une molécule active hydrophile ou un mélange de molécules actives hydrophiles, en particulier l'ectoïne, le d-panthénol, le facteur nmf, l'ascorbate de glucosyle, le phosphate d'ascorbyle sodique, le myo-inositol, l'acétylglucosamine, l'alanylglutamine, l'acétylcystéine, à titre d'ingrédient (o).

12. Produit cosmétique selon l'une quelconque des revendications précédentes, qui contient en outre un agent épaississant ou un mélange d'agents épaississants, en particulier la gomme xanthane, la gomme de cellulose, l'hyaluronane, le glucane, les polymères réticulés d'acrylate de sodium, le polyquaternium 37, la carraghénane, les dérivés alginiques, les dérivés de gomme de guar, à titre d'ingrédient (p).

13. Produit cosmétique selon l'une quelconque des revendications précédentes, dans lequel ledit concentré vésiculaire contient en outre une molécule filmogène ou un mélange de molécules filmogènes, en particulier une protéine de maïs, une protéine d'avoine, une protéine de riz, une protéine de riz hydrolysée, à titre d'ingrédient (q).

14. Produit cosmétique selon l'une quelconque des revendications 1 à 13, comprenant de 0,1 % à 60 % en poids dudit concentré vésiculaire.

15. Procédé pour obtenir un produit cosmétique selon la revendication 1 ou 14, comprenant :

   • la préparation de vésicules biomimétiques végétales ou d'origine végétale ayant des dimensions allant de 0,6 à 1,0 micromètre avec des parois comprenant une phase lipidique avec les ingrédients de l'une quelconque des revendications 1 à 13, par les sous-étapes suivantes :

o la préparation d'une composition contenant un concentré vésiculaire par fonte des parois desdites vésicules jusqu'à l'état fondu de cristal liquide de façon que soit obtenu un système monophasique homogène ;

o l'introduction d'une phase aqueuse dans ladite composition de concentré vésiculaire à une température supérieure à la température de solidification de la phase lipidique des vésicules ;

o le refroidissement de la composition de concentré vésiculaire au-dessous de la température de solidification de la phase lipidique des vésicules jusqu'à obtention d'un matériau ayant une texture pâteuse et ayant des caractéristiques rhéologiques d'un fluide pseudoplastique ou d'un solide viscoélastique avec une distribution des tailles des vésicules comprise entre 0,6 et 1,0 micromètre ;

• la combinaison de la composition concentrée vésiculaire obtenue ayant une texture pâteuse avec de l'eau et d'autres ingrédients cosmétiques jusqu'à obtention d'un produit cosmétique fini contenant un pourcentage en poids souhaité dudit concentré vésiculaire.

16. Procédé pour préparer un produit cosmétique selon la revendication 15, dans lequel ledit concentré vésiculaire est obtenu par les étapes suivantes :

• l'insertion des ingrédients des parois vésiculaires (PHASE A) un à la fois dans un mélangeur chemisé doté d'une turbine (rotor-stator), d'un agitateur mécanique et d'un racleur ;

• l'agitation mécanique du mélange à la température ambiante jusqu'à obtention d'une dispersion lisse et homogène des composants ;

• le chauffage progressif de la PHASE A par incréments de 10°C sur une période de 1 heure jusqu'à la température de 80-100°C afin de permettre la fonte des composants solides, ce qui donne une dispersion translucide légèrement jaunâtre ayant une texture pâteuse ;

• le chauffage de la PHASE A à 60°C avec poursuite de l'agitation mécanique ;

• l'addition progressive dans le mélangeur des composants devant être ajoutés à l'intérieur desdites vésicules (PHASE B), la température étant maintenue constante ;

• une fois que l'addition de la PHASE B est terminée, l'agitation de la composition pendant 30 minutes ;

• l'agitation turbulente du mélange par actionnement de ladite turbine et la réduction progressive de la température jusqu'à la température ambiante, ce qui donne un concentré ayant un aspect pâteux blanc/jaunâtre.

17. Procédé selon la revendication 15, dans lequel lesdits autres ingrédients cosmétiques consistent en des poudres cosmétiques, et il est prévu une étape de séchage finale pour éliminer l'eau et l'obtention d'un produit cosmétique en poudre contenant ladite composition de concentré vésiculaire et des poudres cosmétiques.

Figure 1                                    Figure 2

Figure 3                                    Figure 4

Figure 5 – Production of Collagen I – In-vitro evaluation on cell cultures

Figure 6 – Production of Type I Collagen – Ex-vivo study

Figure 7 – Hydration (AQP3) – In-vitro evaluation on cell cultures

Figure 8 – Expression of AQP3 – Ex-vivo study

Figure 9 – Percentage variation of skin hydration

**Figure 10 – Skin elasticity parameters R0 and R2**

## R0: Average percentage variation of «Firmness»

$$y = -0.0104x^2 + 0.0065x - 0.0306$$

$$R^2 = 0.997$$

$$y = -0.0094x^2 + 0.0034x - 0.0168$$

$$R^2 = 0.98$$

FACE (TREATED) t2weeks          FACE (TREATED) t4weeks

Forearm- Control (Not Treated) t2weeks          Forearm- Control (Not Treated) t4weeks

········ Polin. (FACE (TREATED) t2weeks)          ─ ─ ─ Polin. (FACE (TREATED) t4weeks)

**Figure 11 – Parameter R0 – Percentage variation of skin firmness**

## R2: Average percentage variation of Skin Elasticity

Figure 12 – Parameter R2 – Percentage variation of R2 (Skin Elasticity)

Figure 13 – Examples of face images used for the calculation of the Coefficient of Visibility

## Wrinkles: coefficient of visibility
### Average % variation

Figure 14 – Variation of the coefficient of visibility of wrinkles

## Percentage differences between A2 («stripping» and single application of the formula) and A1 («stripping» only)

Figure 15 – Percentage recovery of TEWL

**Figure 16 – Schematic formation of the vesicular lipid film and observations at electron microscope (CRYO-TEM e TEM)**

**Figure 17 – Scheme elucidating the advantage of micrometric vesicles (on the left) vs. nanometric vesicles (on the right). The lipid content being the same, the nanometric film is provided with a higher number of "defects" and a higher number of percolation paths in the spaces between vesicles**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 101440383 B1

### Non-patent literature cited in the description

- **STROTT** ; **HIGASHI**. Cholesterol sulfate in human physiology: what's it all about?. *Journal of Lipid Research*, 2003, vol. 44, 1268-1278